# EUROPÄISCHE PATENTSCHRIFT

(11) **EP 2 072 083 B1**
(45) Veröffentlichungstag und Bekanntmachung des Hinweises auf die Patenterteilung: **15.02.2012**
(21) Anmeldenummer: 07123731.7
(22) Anmeldetag: 19.12.2007
(51) Int. Cl.: A61Q 5/00, C11B 9/00, C07C 49/76

(54) **Verwendung von 2,4'-Dimethyl-propiophenon als Riechstoff**
Utilisation of 2.4'-dimethyl-propiophenon as an aroma
Utilisation de 2,4'-diméthyl-propiophénone en tant que parfum

(43) Veröffentlichungstag der Anmeldung: 24.06.2009
(73) Patentinhaber: Symrise AG, 37603 Holzminden (DE)
(72) Erfinder: KUHN, Walter, 37603 Holzminden (DE); LAMBRECHT, Stefan, 37619 Hehlen (DE); PANTEN, Johannes, 37671 Höxter (DE); WIEDMANN, Willi, 37639 Bevern (DE)
(74) Vertreter: Eisenführ, Speiser & Partner

(56) Entgegenhaltungen:
- US-A- 4 246 292
- US-A- 4 387 047
- CLAUS AD: "CXII: Zur Kenntniss (sic!) der gemischten fettaromatischen Ketone" JOURNAL FÜR PRAKTISCHE CHEMIE, Bd. 46, 1892, XP002480294 DE

## Beschreibung

Die vorliegende Erfindung betrifft die Verwendung von 2,4'-Dimethyl-propiophenon als Riechstoff in einer Riechstoffkomposition, vor allem zum Vermitteln, Modifizieren und/oder Verstärken der Geruchsnoten blumig, rosig, rosenoxidartig und/oder herbalartig. Ein weiterer Aspekt betrifft Riechstoffkompositionen, die eine wirksame Menge an 2,4'-Dimethyl-propiophenon sowie einen oder mehrere weitere Riechstoffe, bevorzugt Riechstoffe des Fougere-Typs oder des Rosentyps, umfassen, sowie entsprechende Verfahren zur Herstellung dieser Riechstoffkompositionen.

Ein weiterer Aspekt der vorliegenden Erfindung betrifft parfümierte Artikel, welche eine entsprechende Riechstoffkomposition enthalten.

Die Erfindung betrifft zudem die Verwendung von 2,4'-Dimethyl-propiophenon oder entsprechender Riechstoffkompositionen zum Versehen von (a) Haaren oder (b) textilen Fasern mit einem blumigen, rosigen, rosenoxidartigen und/oder herbalartigen Geruch sowie entsprechende Verfahren.

Die Erfindung betrifft ferner bestimmte Verwendungen von 2,4'-Dimethyl-propiophenon als Mittel zum Erhöhen der Substantivität und/oder Retention einer Riechstoffkomposition, die Verwendung als Mittel zum Erhöhen der Komplexstabilität einer Riechstoffkomposition, die Verwendung als Mittel zum Verändern, Maskieren und/oder Vermindern einer medizinischen Geruchsnote in einer Riechstoffkomposition sowie die Verwendung als Mittel zum Erhöhen des über einer tensidhaltigen wässrigen Lösung wahrgenommenen Geruches anderer Riechstoffe.

Zu weiteren Merkmalen der erfindungsgemäßen Riechstoffkompositionen, parfümierten Artikeln, Verfahren und Verwendungen siehe unten.

Trotz einer Vielzahl bereits vorhandener Riechstoffe besteht in der Parfümindustrie auch weiterhin ein genereller Bedarf an neuen Riechstoffen, die über Ihre primären, nämlich geruchlichen, Eigenschaften hinaus zusätzliche positive Sekundäreigenschaften besitzen, wie z.B. eine höhere Stabilität unter bestimmten Anwendungsbedingungen, eine höhere Ausgiebigkeit oder ein besseres Haftungsvermögen, oder aber durch Synergieeffekte mit anderen Riechstoffen zu besseren sensorischen Profilen führen.

Es besteht in der Parfümindustrie auch grundsätzlich ein Bedarf an weiteren Riechstoffen, die sich zur Herstellung von Riechstoffkompositionen oder parfümierten Artikeln eignen. Insbesondere besteht ein Bedarf an Riechstoffen, die durch die oben erwähnten technischen Eigenschaften zu einem erhöhten Nutzen in Riechstoffkompositionen führen. So können z.B. durch den Einsatz von Riechstoffen mit einer höheren Stabilität (besonders gegenüber aggressiven Medien wie Hair Colorationen oder Bleach Mitteln) und einem besseren sensorischen Profil die Einsatzmengen und die Anzahl von Riechstoffen in entsprechenden Formulierungen optimiert und/oder minimiert werden, was zu einer nachhaltigen Ressourcenschonung beim Parfümieren von Konsum- und Verbrauchsgütern führt.

Daher besteht in der Parfümindustrie insbesondere ein Bedarf an weiteren Riechstoffen mit besseren sensorischen Profilen und höherer Stabilität, vor allem gegenüber aggressiven Medien.

Dabei besteht in der Parfümindustrie insbesondere ein Bedarf an Riechstoffen mit blumigen, herbalen (krautigen) bzw. herbalartigen Noten. Hierunter ist im Rahmen des vorliegenden Textes ein Geruch zu verstehen, der ähnlich ist wie der Geruch des natürlich vorkommenden Rosenöls bzw. wie der seiner Bestandteile, aber eine zusätzliche herbale Note besitzt. Der Geruch von Rosenoxid als Bestandteil von natürlichem Bulgarian Rose Oil z.B. wird in der Literatur wie folgt beschrieben: blumiggeraniumartig, grün, hart (Quelle: S. Arctander, Perfume and Flavor Chemicals, Vol. I und II, Montclair, N. J., 1969, Selbstverlag , Nr. 2809.). Der parfümistische Begriff "herbal" wird genauer als "grasgrün, würzig und medizinisch" beschrieben (Quelle: P.M. Müller, D. Lamparsky, Perfumes - Art, Science, and Technology, 1991, Elsevier Applied Science, London, New York, S. 284).

Primäre Aufgabe der vorliegenden Erfindung war es, Riechstoffkompositionen enthaltend einen Rosen-Riechstoff mit zusätzlichem herbalen Charakter anzugeben. Bevorzugt sollten Riechstoffkompositionen enthaltend Riechstoffe mit einer rosenoxidartigen und herbalartigen Geruchsnote angegeben werden. Ferner sollten diese Riechstoffe bzw. die Riechstoffkompositionen die oben genannten technischen Eigenschaften bzw. Vorteile aufweisen.

Des Weiteren sollten mit der vorliegenden Erfindung entsprechende vorteilhafte Verwendungen des Riechstoffs bereitgestellt werden. Weitere Aufgabenstellungen, die der vorliegenden Erfindung zu Grunde liegen, ergeben sich aus den nachfolgenden Ausführungen und den beigefügten Patentansprüchen.

Im Rahmen der erfindungsgemäßen Aufgabenstellungen sollte vor allem darauf abgezielt werden, entsprechende Riechstoffe in Riechstoffkompositionen des Fougere-Typs einzusetzen.

Die vorliegende Erfindung betrifft gemäß einem primären Aspekt die Verwendung von 2,4'-Dimethyl-propiophenon als Riechstoff in einer Riechstoffkomposition. 2,4'-Dimethyl-propiophenon eignet sich besonders gut zur Verwendung als Riechstoff in einer Riechstoffkomposition zum Vermitteln, Modifizieren und/oder Verstärken einer, zwei, drei oder sämtlicher der Geruchsnoten blumig, rosig, rosenoxidartig und herbalartig, vorzugsweise beider der Geruchsnoten rosenoxidartig und herbalartig. Den im Rahmen des vorliegenden Textes beschriebenen Geruchsnoten sind auch die entsprechenden Noten eines Nachgeruchs zuzuordnen. D.h. 2,4'-Dimethyl-propiophenon eignet sich insbesondere auch zur Verwendung als Riechstoff in einer Riechstoffkomposition zum Vermitteln, Modifizieren und/oder Verstärken einer, mehrerer oder sämtlicher der oben genannten Geruchsnoten und/oder eines Nachgeruchs mit einer oder mehrerer der oben genannten Noten. Den in den unten ausgeführten weiteren Aspekten der vorliegenden Erfindung beschriebenen Geruchsnoten sind ebenfalls die entsprechenden Noten eines Nachgeruchs zuzuordnen.

Wie anhand der folgenden Ausführungen deutlich wird, war es besonders überraschend, dass sich 2,4'-Dimethyl-propiophenon als Riechstoff im Sinne der oben beschriebenen Verwendung(en) eignet.

Die Strukturformel von 2,4'-Dimethyl-propiophenon ist nachfolgend wiedergegeben:

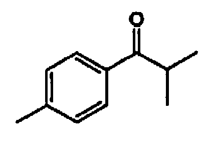

2,4'-Dimethyl-propiophenon (CAS-Nummer: 50390-51-7) ist bereits bekannt.

Ad. Claus.: "CXII: Zur Kenntniss der gemischten fettaromatischen Ketone", Journal für Praktische Chemie, Bd. 46, 1892, beschreibt Isopropyl-Tolyketon (2.4'-Dimethyl-propiophenon) als eine Flüssigkeit von aromatischem Geruch und bitterem Geschmack.

In DE 2808817 werden neue Acyl-polyalkyl-indan-Verbindungen beschrieben, sowie deren Verwendung als Grundstoff für Riechstoffe, entsprechende Riechstoffkompositionen, parfümierte Stoffe und parfümierte Artikel. Dort wird im Rahmen eines Verfahrens zur Herstellung bestimmter Verbindungen ein Keton einer allgemeinen Strukturformel, wobei 2,4'-Dimethyl-propiophenon unter diese Strukturformel fällt, beschrieben, welches in bekannter Weise zum entsprechenden Alkohol reduziert wird, um dann in mehreren Schritten zu dem gewünschten Polyalkyl-indan umgesetzt zu werden. Für die synthetisierten Duftstoffe wird ein Moschus-Charakter beschrieben. In DE 10236918 wird ebenfalls lediglich ein Keton einer allgemeinen Strukturformel als Ausgangsubstanz für die Synthese entsprechender Riechstoffe beschrieben, wobei 2,4'-Dimethyl-propiophenon unter diese Strukturformel fällt. Dabei wird ein Verfahren zur Herstellung von Alkylphenylcarbinolen angegeben, wobei entsprechende Alkylphenylketone in Gegenwart von Nickel-Katalysatoren und basischen Zusätzen hydriert werden, um die gewünschten Alkylphenylcarbinole herzustellen.

In JP 07173095 wird in allgemeiner Form ein neuartiges Verfahren für die Darstellung von substituierten acylierten Aromaten durch Umsetzung des Acylierungsreagenzes mit dem entsprechenden Aromaten in der Gasphase beschrieben.

In DE 19501303 wird erneut in lediglich allgemeiner Form die Verwendung von Arylketonen als Ausgangsmaterial für Haarfärbemittel beschrieben.

In den genannten Dokumenten wird zum sensorischen Charakter von 2,4'-Dimethyl-propiophenon selbst nichts ausgeführt.

Wie bereits erwähnt, eignet sich 2,4'-Dimethyl-propiophenon als Riechstoff in Riechstoffkompositionen zum Vermitteln, Modifizieren und/oder Verstärken einer, zwei, drei oder sämtlicher der Geruchsnoten blumig, rosig, rosenoxidartig und herbalartig. Besonders geeignet ist 2,4'-Dimethyl-propiophenon in Riechstoffkompositionen für eine entsprechende Verwendung zum Vermitteln, Modifizieren und/oder Verstärken beider der Geruchsnoten rosenoxidartig und herbalartig. Die Tatsache, dass diese Verbindung einen ausdrucksstarken rosenoxidartigen und herbalartigen Geruch aufweist ist besonders überraschend, da es sich bei der Verbindung nicht, wie beispielsweise bei Rosenoxid selbst, um einen zyklischen Ether, sondern um einen acylierten Aromaten handelt. Üblicherweise führt ein Wechsel der Funktionalitäten auch bei sonst strukturell ähnlichen Verbindungen zu deutlich unterschiedlichen olfaktorischen Eigenschaften. Auch besteht keine Strukturähnlichkeit mit typisch herbal-artigen oder herbal-ähnlichen Riechstoffen wie z.B. D-Carvon (= (+)-Carvon).
Rosenoxid: D-Carvon:

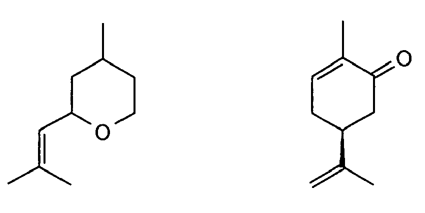

Überraschend ist auch, dass strukturell ähnliche acylierte Aromaten geruchlich anders beschrieben werden. Die nachfolgende Aufzählung zeigt exemplarisch ausgewählte Duftbeschreibungen strukturell ähnlicher acylierter Aromaten (Quelle: S. Arctander, Perfume and Flavor Chemicals, Vol. I und II, Montclair, N. J., 1969, Selbstverlag oder K. Bauer, D. Garbe und H. Surburg, Common Fragrance and Flavor Materials, 4rd. Ed., Wiley-VCH, Weinheim 2001):
- p-Methylacetophenon ist ein wichtiger Riechstoff, der einen blumigen Geruch nach Mimosa, Cumarin und Bittermandel besitzt. (S. Arctander, Perfume and Flavour Chemicals, No. 1895, 1969, Montclair, N.J. (USA)). 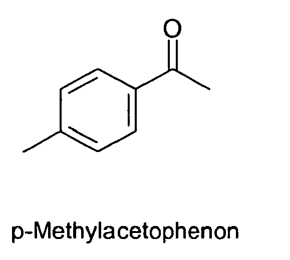
- p-Ethylacetophenon ist ein Riechstoff, der einen süßen, warmen, stechend blumigen Geruch mit einem krautigen-balsamischen Unterton besitzt. (S. Arctander, Perfume and Flavour Chemicals, No. 1139, 1969, Montclair, N.J. (USA)). 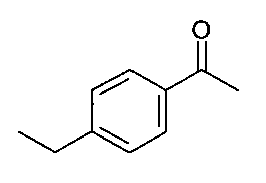 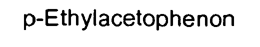
- p-Isopropylacetophenon ist ein Riechstoff, der einen trocken krautigen und holzigen Geruch besitzt. Im Geschmackstoffbereich wird p-Isopropylacetophenon in Honig- und Fruchtaromen verwendet. (S. Arctander, Perfume and Flavour Chemicals, No. 17, 1969, Montclair, N.J. (USA)). 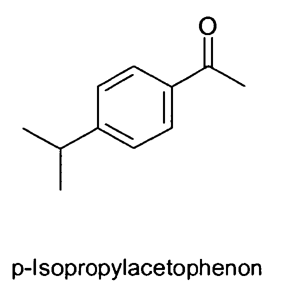
- p-Isobutylacetophenon ist ein Riechstoff, der p-Methylacetophenon ähnelt und sich durch eine holzig-heuartige Note und hohe Haftfestigkeit auszeichnet. (S. Arctander, Perfume and Flavour Chemicals, No. 386, 1969, Montclair, N.J. (USA)).

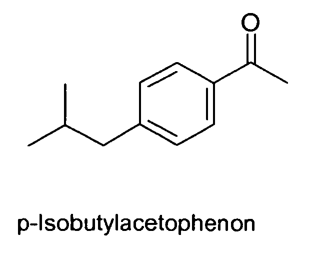

Wie beim Vergleich ersichtlich wird, zeigt keiner der acylierten Aromaten eine rosenoxidartige und herbalartige Geruchsnote. Es war somit besonders überraschend, dass der erfindungsgemäß zu verwendende acylierte Aromat eine rosenoxidartige und herbalartige Geruchsnote aufweist.

2,4'-Dimethyl-propiophenon eignet sich für die erfindungsgemäße Verwendung nicht nur aufgrund der beschriebenen Geruchsnoten sondern auch aufgrund seiner olfaktorischen Eigenschaften, sowie der stofflichen Eigenschaften, wie Löslichkeit in gängigen kosmetischen Lösungsmitteln, Kompatibilität mit den gängigen weiteren Bestandteilen derartiger Produkte, etc.. Zudem unterstreicht die toxikologische Unbedenklichkeit dieses Riechstoffes die besondere Eignung der Verbindung für die weiter oben und im Folgenden genannten Einsatzzwecke. Weitere positive Eigenschaften der erfindungsgemäß zu verwendenden Verbindung sowie entsprechender Mischungen umfassend diese Verbindung werden im Rahmen erfindungsgemäßer Verwendungen weiter unten beschrieben.

Aufgrund der genannten olfaktorischen und stofflichen Eigenschaften eignet sich 2,4'-Dimethyl-propiophenon besonders gut zur Verwendung als Bestandteil einer Riechstoffkomposition des Fougère-Typs oder des Rosen-Typs. Vorzugsweise wird 2,4'-Dimethyl-propiophenon in diesen Riechstoffkompositionen gemäß einer oben beschriebenen erfindungsgemäßen Verwendung eingesetzt.

Rosenoxid- und herbalartige Geruchsnoten werden in vielfältigen Parfümkompositionen eingesetzt, insbesondere z.B. auch in Fougere- oder Rosenduftthemen. Das (in Parfümkompositionen) überraschenderweise blumige, rosige, rosenoxidartige und herbalartige, insbesondere rosenoxidartig und herbalartige Geruchsprofil von 2,4'-Dimethyl-propiophenon trägt daher dazu bei, dass dieser Riechstoff für entsprechende Verwendungen in diesen Duftthemen besonders geeignet ist. Vor allem im Zusammenhang mit Riechstoffkompositionen des Fougere-Typs wird eine Vielzahl der positiven Eigenschaften von 2,4'-Dimethyl-propiophenon besonders deutlich. Das weiter unten beschriebene Beispiel eines Fougére-Duftthemas (siehe Beispiel 2) demonstriert in anschaulicher Weise den olfaktorischen Effekt und die positiven Eigenschaften von 2,4'-Dimethyl-propiophenon. Weitere Vorzüge der vorliegenden Erfindung finden sich somit weiter unten.

Wie bereits erwähnt findet der Riechstoff 2,4'-Dimethyl-propiophenon erfindungsgemäß vorzugsweise Verwendung als Bestandteil von Riechstoffkompositionen des Fougere-Typs oder des Rosen-Typs, vorzugsweise nach einer oben beschriebenen Verwendung. Diese bevorzugten Ausführungsformen sollen die vorliegende Erfindung in ihren Aspekten jedoch in keiner Weise einschränken. Der erfindungsgemäß zu verwendende Riechstoff kann vielmehr gemäß einem weiteren Aspekt der vorliegenden Erfindung ganz allgemein in Riechstoffkompositionen enthalten sein. Dementsprechend betrifft die vorliegende Erfindung auch eine Riechstoffkomposition, die 2,4'-Dimethyl-propiophenon sowie einen, zwei, drei oder mehr weitere Riechstoffe enthält, wobei das Gewichtsverhältnis der Gesamtmenge an 2,4'-Dimethyl-propiophenon zu der Gesamtmenge an weiteren Riechstoffen im Bereich von 1:1000 bis 1:0,5 liegt, vorzugsweise im Bereich von 1:700 bis 1:1, besonders bevorzugt im Bereich von 1:500 bis 1:10.

Es ist dabei besonders bevorzugt, wenn in einer besagten Riechstoffkomposition eine Menge an 2,4'-Dimethyl-propiophenon enthalten ist, die ausreicht, eine, zwei, drei oder sämtliche der Geruchsnoten, blumig, rosig, rosenoxidartig und herbalartig zu vermitteln, zu modifizieren und/oder zu verstärken (organoleptisch wirksame Menge). Vorzugsweise gilt dies für eine Kombination der Geruchsnoten rosenoxidartig und herbalartig.

Wie sich im Rahmen eigener Untersuchungen herausstellte, sind diejenigen der oben beschriebenen Riechstoffkompositionen für die Zwecke der vorliegenden Erfindung besonders geeignet, welche eine Menge an 2,4'-Dimethyl-propiophenon im Bereich von 0,01 bis 65 Gew.-%, vorzugsweise von etwa 0,1 bis etwa 50 Gew.-%, vorzugsweise von etwa 0,5 bis etwa 30 Gew.-% und besonders bevorzugt von etwa 0,5 bis etwa 25 Gew.-%, umfassen, bezogen auf die Gesamtmenge der Riechstoffkomposition.

In Mischung mit anderen Riechstoffen vermag das erfindungsgemäß einzusetzende 2,4'-Dimethyl-propiophenon demnach bereits in geringen Dosierungen der Riechstoffkomposition eine oben beschriebene gewünschte Geruchsnote zu verleihen.

Wie bereits weiter oben erwähnt ist es ferner bevorzugt, wenn in den (zuvor genannten bzw. weiter unten erläuterten) erfindungsgemäßen Riechstoffkompositionen eine Menge an 2,4'-Dimethyl-propiophenon enthalten ist, die ausreicht, um eine, zwei, drei oder sämtliche der Geruchsnoten, blumig, rosig, rosenoxidartig und herbalartig und/oder eine, zwei, drei oder sämtliche der Nachgeruchsnoten blumig, rosig, rosenoxidartig und herbalartig zu vermitteln, zu modifizieren und/oder zu verstärken.

Besonders bevorzugt sind Riechstoffkompositionen oder parfümierte Artikel, die eine Menge an 2,4'-Dimethyl-propiophenon enthalten, die ausreicht, um eine rosenoxid- und herbalartige Geruchsnote zu vermitteln, zu modifizieren und/oder zu verstärken.

Wie beschrieben, umfassen die erfindungsgemäßen Riechstoffkompositionen einen oder mehrere weitere Riechstoffe. Gemäß einem weiteren Aspekt der vorliegenden Erfindung sind dabei erfindungsgemäße Riechstoffkompositionen besonders bevorzugt, welche zumindest einen weiteren Riechstoff, vorzugsweise 2, 3, 4, 5, 6, 7, 8 oder mehr weitere Riechstoffe umfassen, wobei der eine oder die mehreren weiteren Riechstoffe ausgewählt sind aus der Gruppe bestehend aus:
Alpha-Hexylzimtaldehyd, 2-Phenoxyethylisobutyrat (Phenirat¹), Dihydromyrcenol (2,6-Dimethyl-7-octen-2-ol), Methyldihydrojasmonat (vorzugsweise mit einem Gehalt an cis-Isomerem von mehr als 60 Gew.-%) (Hedione⁹, Hedione HC⁹), 4,6,6,7,8,8-Hexamethyl-1,3,4,6,7,8-hexahydrocyclopenta[g]benzopyran (Galaxolid³), Tetrahydrolinalool (3,7-Dimethyloctan-3-ol), Ethyllinalool, Benzylsalicylat, 2-Methyl-3-(4-tert-butylphenyl)propanal (Lilial²), Zimtalkohol, 4,7-Methano-3a,4,5,6,7,7a-hexahydro-5-indenylacetat und/oder 4,7-Methano-3a,4,5,6,7,7a-hexahydro-6-indenylacetat (Herbaflorat¹), Citronellol, Vanillin, Linalylacetat, Styrolylacetat (1-Phenylethylacetat), Octahydro-2,3,8,8-tetramethyl-2-acetonaphthon und/oder 2-Acetyl-1,2,3,4,6,7,8-octahydro-2,3,8,8-tetramethylnaphtalin (Iso E Super³), Hexylsalicylat, 4-tert.-Butylcyclohexylacetat (Oryclone¹), 2-tert.-Butylcyclohexylacetat (Agrumex HC¹), alphalonon (4-(2,2,6-Trimethyl-2-cyclohexen-1-yl)-3-buten-2-on), n-alpha-Methylionon, alpha-Isomethylionon, Coumarin, Terpinylacetat, 2-Phenylethylalkohol, 4-(4-Hydroxy-4-methylpentyl)-3-cyclohexencarboxaldehyd (Lyral³), alpha-Amylzimtaldehyd, Ethylenbrassylat, (E)- und/oder (Z)-3-Methylcyclopentadec-5-enon (Muscenon⁹), 15-Pentadec-11-enolid und/oder 15-Pentadec-12-enolid (Globalide¹), 15-Cyclopentadecanolid (Macrolide¹), 1-(5,6,7,8-Tetrahydro-3,5,5,6,8,8-hexamethyl-2-naphthalenyl)ethanon (Tonalid¹⁰),2-Isobutyl-4-methyltetrahydro-2*H*-pyran-4-ol (Florol⁹), 2-Ethyl-4- (2,2,3-trimethyl-3-cyclopenten-1-yl)-2-buten-1-ol (Sandolen¹), cis-3-Hexenylacetat, trans-3-Hexenylacetat, trans-2,cis-6-Nonadienol, 2,4-dimethyl-3-cyclohexencarboxaldehyd (Vertocitral¹), 2,4,4,7-Tetramethyl-oct-6-en-3-on (Claritone¹), 2,6-Dimethyl-5-hepten-1-al (Melonal²), Borneol, 3-(3-Isopropylphenyl)butanal (Florhydral²), 2-Methyl-3-(3,4-methylendioxyphenyl)propanal (Helional³), 3-(4-Ethylphenyl)-2,2-dimethylpropanal (Florazon¹), 7-Methyl-2*H*-1,5-benzodioxepin-3(4*H*)-on (Calone 1951⁵), 3,3,5-Trimethylcyclohexylacetat (vorzugsweise mit einem Gehalt an cis-Isomeren von 70 Gew.-%) oder mehr und 2,5,5-Trimethyl-1,2,3,4,4a,5,6,7-octahydronaphthalin-2-ol (Ambrinol S¹). Die vorangehend genannten Riechstoffe werden im Rahmen der vorliegenden Erfindung demnach bevorzugt mit 2,4'-Dimethyl-propiophenon kombiniert. Sofern vorstehend Handelsnamen angegeben sind, beziehen sich diese auf folgende Quellen:
¹ Handelsname Symrise GmbH, Deutschland;
² Handelsname Givaudan AG, Schweiz;
³ Handelsname International Flavors & Fragrances Inc., USA;
⁵ Handelsname Danisco Seillans S.A., Frankreich;
⁹ Handelsname Firmenich S.A., Schweiz;
¹⁰ Handelsname PFW Aroma Chemicals B.V., Niederlande.

Weitere Riechstoffe, mit denen das erfindungsgemäß zu verwendende 2,4'-Dimethyl-propiophenon vorteilhaft kombiniert werden kann, finden sich z.B. in S. Arctander, Perfume and Flavor Chemicals, Vol. I und II, Montclair, N. J., 1969, Selbstverlag oder K. Bauer, D. Garbe und H. Surburg, Common Fragrance and Flavor Materials, 4rd. Ed., Wiley-VCH, Weinheim 2001. Im einzelnen seien genannt:
Extrakte aus natürlichen Rohstoffen wie Etherische Öle, Concretes, Absolues, Resine, Resinoide, Balsame, Tinkturen wie z.B. Ambratinktur; Amyrisöl; Angelicasamenöl; Angelicawurzelöl; Anisöl; Baldrianöl; Basilikumöl; Baummoos-Absolue; Bayöl; Beifußöl; Benzoeresin; Bergamotteöl; Bienenwachs-Absolue; Birkenteeröl; Bittermandelöl; Bohnenkrautöl; Buccoblätteröl; Cabreuvaöl; Cadeöl; Calmusöl; Campheröl; Canangaöl; Cardamomenöl; Cascarillaöl; Cassiaöl; Cassie-Absolue; Castoreum-absolue; Cedernblätteröl; Cedernholzöl; Cistusöl; Citronellöl; Citronenöl; Copaivabalsam; Copaivabalsamöl; Corianderöl; Costuswurzelöl; Cuminöl; Cypressenöl; Davanaöl; Dillkrautöl; Dillsamenöl; Eau de brouts-Absolue; Eichenmoos-Absolue; Elemiöl; Estragonöl; Eucalyptus-citriodora-Öl; Eucalyptusöl; Fenchelöl ; Fichtennadelöl; Galbanumöl; Galbanumresin; Geraniumöl; Grapefruitöl; Guajakholzöl; Gurjunbalsam; Gurjunbalsamöl; Helichrysum-Absolue; Helichrysumöl; Ingweröl; Iriswurzel-Absolue; Iriswurzelöl; Jasmin-Absolue; Kalmusöl; Kamillenöl blau; Kamillenöl römisch; Karottensamenöl; Kaskarillaöl; Kiefernadelöl; Krauseminzöl; Kümmelöl; Labdanumöl; Labdanum-Absolue; Labdanumresin; Lavandin-Absolue; Lavandinöl ; Lavendel-Absolue; Lavendelöl; Lemongrasöl; Liebstocköl; Limetteöl destilliert; Limetteöl gepreßt; Linaloeöl; Litsea-cubeba-Öl; Lorbeerblätteröl; Macisöl; Majoranöl; Mandarinenöl; Massoirindenöl; Mimosa-Absolue; Moschuskörneröl; Moschustinktur; Muskateller-Salbei-Öl; Muskatnußöl; Myrrhen-Absolue; Myrrhenöl; Myrtenöl; Nelkenblätteröl; Nelkenblütenöl; Neroliöl; Olibanum-Absolue; Olibanumöl; Opopanaxöl; Orangenblüten-Absolue; Orangenöl; Origanumöl; Palmarosaöl; Patchouliöl; Perillaöl; Perubalsamöl; Petersilienblätteröl; Petersiliensamenöl; Petitgrainöl; Pfefferminzöl; Pfefferöl; Pimentöl; Pineöl; Poleyöl; Rosen-Absolue; Rosenholzöl; Rosenöl; Rosmarinöl; Salbeiöl dalmatinisch; Salbeiöl spanisch; Sandelholzöl; Selleriesamenöl; Spiklavendelöl; Sternanisöl; Styraxöl; Tagetesöl; Tannennadelöl; Tea-tree-Öl; Terpentinöl; Thymianöl; Tolubalsam; Tonka-Absolue; Tuberosen-Absolue; Vanilleextrakt; Veilchenblätter-Absolue; Verbenaöl; Vetiveröl; Wacholderbeeröl; Weinhefenöl; Wermutöl; Wintergrünöl; Ylangöl; Ysopöl; Zibet-Absolue; Zimtblätteröl; Zimtrindenöl sowie Fraktionen davon, bzw. daraus isolierten Inhaltsstoffen;
Einzel-Riechstoffe aus der Gruppe der Kohlenwasserstoffe, wie z.B. 3-Caren; alpha-Pinen; beta-Pinen; alpha-Terpinen; gamma-Terpinen; p-Cymol; Bisabolen; Camphen; Caryophyllen; Cedren; Farnesen; Limonen; Longifolen; Myrcen; Ocimen; Valencen; (E,Z)-1,3,5-Undecatrien; Styrol; Diphenylmethan;
der aliphatischen Alkohole wie z.B. Hexanol; Octanol; 3-Octanol; 2,6-Dimethylheptanol; 2-Methyl-2-heptanol; 2-Methyl-2-octanol; (E)-2-Hexenol; (E)- und (Z)-3-Hexenol; 1-Octen-3-ol; Gemisch von 3,4,5,6,6-Pentamethyl-3/4-hepten-2-ol und 3,5,6,6-Tetramethyl-4-methyleneheptan-2-ol; (E,Z)-2,6-Nonadienol; 3,7-Dimethyl-7-methoxyoctan-2-ol; 9-Decenol; 10-Undecenol; 4-Methyl-3-decen-5-ol;
der aliphatischen Aldehyde und deren Acetale wie z.B. Hexanal; Heptanal; Octanal; Nonanal; Decanal; Undecanal; Dodecanal; Tridecanal; 2-Methyloctanal; 2-Methylnonanal; (E)-2-Hexenal; (Z)-4-Heptenal; 2,6-Dimethyl-5-heptenal; 10-Undecenal; (E)-4-Decenal; 2-Dodecenal;2,6,10-Trimethyl-9-undecenal; 2,6,10-Trimethyl-5,9-undecadienal; Heptanaldiethylacetal; 1,1-Dimethoxy-2,2,5-trimethyl-4-hexen; Citronellyloxyacetaldehyd; 1-(1-Methoxy-propoxy)-(E/Z)-3-hexen;
der aliphatischen Ketone und deren Oxime wie z.B. 2-Heptanon; 2-Octanon; 3-Octanon; 2-Nonanon; 5-Methyl-3-heptanon; 5-Methyl-3-heptanonoxim; 2,4,4,7-Tetramethyl-6-octen-3-on; 6-Methyl-5-hepten-2-on;
der aliphatischen schwefelhaltigen Verbindungen wie z.B. 3-Methylthio-hexanol; 3-Methylthiohexylacetat; 3 -Mercaptohexanol; 3-Mercaptohexylacetat; 3-Mercaptohexylbutyrat; 3-Acetylthiohexylacetat; 1-Menthen-8-thiol;
der aliphatischen Nitrile wie z.B. 2-Nonensäurenitril; 2-Undecensäurenitril; 2-Tridecensäurenitril; 3,12-Tridecadiensäurenitril; 3,7-Dimethyl-2,6-octadien-säurenitril; 3,7-Dimethyl-6-octensäurenitril;
der Ester von aliphatischen Carbonsäuren wie z.B. (E)- und (Z)-3-Hexenylformiat; Ethylacetoacetat; Isoamylacetat; Hexylacetat; 3,5,5-Trimethylhexylacetat; 3-Methyl-2-butenylacetat; (E)-2-Hexenylacetat; (E)- und (Z)-3-Hexenylacetat; Octylacetat; 3-Octylacetat; 1-Octen-3-ylacetat; Ethylbutyrat; Butylbutyrat; Isoamylbutyrat; Hexylbutyrat; (E)- und (Z)-3-Hexenyl-isobutyrat; Hexylcrotonat; Ethylisovalerianat; Ethyl-2-methylpentanoat; Ethylhexanoat; Allylhexanoat; Ethylheptanoat; Allylheptanoat; Ethyloctanoat; Ethyl-(E,Z)-2,4-decadienoat; Methyl-2-octinat; Methyl-2-noninat; Allyl-2-isoamyloxyacetat; Methyl-3,7-dimethyl-2,6-octadienoat;4-Methyl-2-pentyl-crotonat;
der acyclischen Terpenalkohole wie z.B. Citronellol; Geraniol; Nerol; Linalool; Lavadulol; Nerolidol; Farnesol; Tetrahydrolinalool; Tetrahydrogeraniol; 2,6-Dimethyl-7-octen-2-ol; 2,6-Dimethyloctan-2-ol; 2-Methyl-6-methylen-7-octen-2-ol; 2,6-Dimethyl-5,7-octadien-2-ol; 2,6-Dimethyl-3,5-octadien-2-ol; 3,7-Dimethyl-4,6-octadien-3-ol; 3,7-Dimethyl-1,5,7-octatrien-3-ol 2,6-Dimethyl-2,5,7-octatrien-1-ol; sowie deren Formiate, Acetate, Propionate, Isobutyrate, Butyrate, Isovalerianate, Pentanoate, Hexanoate, Crotonate, Tiglinate und 3-Methyl-2-butenoate;
der acyclischen Terpenaldehyde und -ketone wie z.B. Geranial; Neral; Citronellal; 7-Hydroxy-3,7-dimethyloctanal; 7-Methoxy-3,7-dimethyloctanal; 2,6,1 0-Trimethyl-9-undecenal; Geranylaceton; sowie die Dimethyl- und Diethylacetale von Geranial, Neral, 7-Hydroxy-3,7-dimethyloctanal;
der cyclischen Terpenalkohole wie z.B. Menthol; Isopulegol; alpha-Terpineol; Terpinenol-4; Menthan-8-ol; Menthan-1-ol; Menthan-7-ol; Borneol; Isoborneol; Linalooloxid; Nopol; Cedrol; Ambrinol; Vetiverol; Guajol; sowie deren Formiate, Acetate, Propionate, Isobutyrate, Butyrate, Isovalerianate, Pentanoate, Hexanoate, Crotonate, Tiglinate und 3-Methyl-2-butenoate;
der cyclischen Terpenaldehyde und -ketone wie z.B. Menthon; Isomenthon ; 8-Mercaptomenthan-3-on ; Carvon; Campher; Fenchon; alpha-Ionon; beta-Ionon; alphan-Methylionon; beta-n-Methylionon; alpha-Isomethylionon; beta-Isomethylionon; alpha-Iron; alpha-Damascon; beta-Damascon; beta-Damascenon; delta-Damascon; gamma-Damascon; 1-(2,4,4-Trimethyl-2-cyclohexen-1-yl)-2-buten-1-on; 1,3,4,6,7,8a-Hexahydro-1,1,5,5-tetramethyl-2H-2,4a-methanonaphthalen-8(5H)-on;2-Methyl-4-(2,6,6-trimethyl-1-cyclohexen-1-yl)-2-butenal; Nootkaton; Dihydronootkaton; 4,6,8-Megastigmatrien-3-on; alpha-Sinensal ; beta-Sinensal ; acetyliertes Cedernholzöl (Methylcedrylketon);
der cyclischen Alkohole wie z.B. 4-tert.-Butylcyclohexanol; 3,3,5-Trimethylcyclohexanol; 3-Isocamphylcyclohexanol; 2,6,9-Trimethyl-Z2,Z5,E9-cyclododecatrien-1-ol; 2-Isobutyl-4-methyltetrahydro-2H-pyran-4-ol;
der cycloaliphatischen Alkohole wie z.B. alpha,3,3-Trimethylcyclohexylmethanol;1-(4-Isopropylcyclohexyl)ethanol; 2-Methyl-4-(2,2,3-trimethyl-3-cyclopent-1-yl)butanol; 2-Methyl-4-(2,2,3-trimethyl-3-cyclopent-1-yl)-2-buten-1-ol; 2-Ethyl-4-(2,2,3-trimethyl-3-cyclopent-1-yl)-2-buten-1-ol; 3-Methyl-5-(2,2,3-trimethyl-3-cyclopent-1-yl)-pentan-2-ol; 3-Methyl-5-(2,2,3-trimethyl-3-cyclopent-1-yl)-4-penten-2-ol; 3,3-Dimethyl-5-(2,2,3-trimethyl-3-cyclopent-1-yl)-4-penten-2-ol; 1-(2,2,6-Trimethylcyclohexyl)pentan-3-ol; 1-(2,2,6-Trimethylcyclohexyl)hexan-3-ol;
der cyclischen und cycloaliphatischen Ether wie z.B. Cineol; Cedrylmethylether; Cyclododecylmethylether;1,1-Dimethoxycyclododecan; (Ethoxymethoxy)cyclododecan; alpha-Cedrenepoxid; 3a,6,6,9a-Tetramethyldodecahydronaphtho[2,1-b]furan; 3a-Ethyl-6,6,9a-trimethyldodecahydronaphtho[2,1-b]furan; 1,5,9-Trimethyl-13-oxabicyclo[10.1.0]trideca-4,8-dien; Rosenoxid; 2-(2,4-Dimethyl-3-cyclohexen-1-yl)-5-methyl-5-(1-methylpropyl)-1,3-dioxan;
der cyclischen und makrocyclischen Ketone wie z.B. 4-tert.-Butylcyclohexanon; 2,2,5-Trimethyl-5-pentylcyclopentanon; 2-Heptylcyclopentanon; 2-Pentylcyclopentanon; 2-Hydroxy-3-methyl-2-cyclopenten-1-on; 3-Methyl-cis-2-penten-1-yl-2-cyclopenten-1-on; 3-Methyl-2-pentyl-2-cyclopenten-1-on; 3-Methyl-4-cyclopentadecenon; 3-Methyl-5-cyclopentadecenon; 3-Methylcyclopentadecanon; 4-(1-Ethoxyvinyl)-3,3,5,5-tetramethylcyclohexanon; 4-tert.-Pentylcyclohexanon; 5-Cyclohexadecen-1-on; 6,7-Dihydro-1,1,2,3,3-pentamethyl-4(5H)-indanon; 8-Cyclohexadecen-1-on; 7-Cyclohexadecen-1-on; (7/8)-Cyclohexadecen-1-on; 9-Cycloheptadecen-1-on; Cyclopentadecanon; Cyclohexadecanon;
der cycloaliphatischen Aldehyde wie z.B. 2,4-Dimethyl-3-cyclohexencarbaldehyd; 2-Methyl-4-(2,2,6-trimethyl-cyclohexen-1-yl)-2-butenal; 4-(4-Hydroxy-4-methylpentyl)-3-cyclohexencarbaldehyd; 4-(4-Methyl-3-penten-1-yl)-3-cyclohexencarbaldehyd;
der cycloaliphatischen Ketone wie z.B. 1-(3,3-Dimethylcyclohexyl)-4-penten-1-on; 2,2-Dimethyl-1-(2,4-dimethyl-3-cyclohexen-1-yl)-1-propanon; 1-(5,5-Dimethyl-1-cyclohexen-1-yl)-4-penten-1-on; 2,3,8,8-Tetramethyl-1,2,3,4,5,6,7,8-octahydro-2-naphtalenylmethylketon; Methyl-2,6,10-trimethyl-2,5,9-cyclododecatrienylketon; tert.-Butyl-(2,4-dimethyl-3-cyclohexen-1-yl)keton;
der Ester cyclischer Alkohole wie z.B. 2-tert-Butylcyclohexylacetat; 4-tert-Butylcyclohexylacetat; 2-tert-Pentylcyclohexylacetat; 4-tert-Pentylcyclohexylacetat; 3,3,5-Trimethylcyclohexylacetat; Decahydro-2-naphthylacetat;2-Cyclopentylcyclopentylcrotonat; 3-Pentyltetrahydro-2H-pyran-4-ylacetat; Decahydro-2,5,5,8a-tetramethyl-2-naphthylacetat; 4,7-Methano-3a,4,5,6,7,7a-hexahydro-5, bzw. 6-indenylacetat; 4,7-Methano-3a,4,5,6,7,7a-hexahydro-5, bzw. 6-indenylpropionat; 4,7-Methano-3a,4,5,6,7,7a-hexahydro-5, bzw. 6-indenylisobutyrat; 4,7-Methanooctahydro-5, bzw. 6-indenylacetat;
der Ester cycloaliphatischer Alkohole wie z.B.1-Cyclohexylethylcrotonat;
der Ester cycloaliphatischer Carbonsäuren wie z.B. Allyl-3-cyclohexylpropionat; Allylcyclohexyloxyacetat; cis- und trans-Methyldihydrojasmonat; cis- und trans-Methyljasmonat; Methyl-2-hexyl-3-oxocyclopentancarboxylat; Ethyl-2-ethyl-6,6-dimethyl-2-cyclohexencarboxylat; Ethyl-2,3,6,6-tetramethyl-2-cyclohexencarboxylat; Ethyl-2-methyl-1,3-dioxolan-2-acetat;
der araliphatischen Alkohole wie z.B. Benzylalkohol; 1-Phenylethylalkohol; 2-Phenylethylalkohol; 3-Phenylpropanol; 2-Phenylpropanol; 2-Phenoxyethanol; 2,2-Dimethyl-3-phenylpropanol; 2,2-Dimethyl-3-(3-methylphenyl)propanol; 1,1-Dimethyl-2-phenylethylalkohol; 1,1-Dimethyl-3-phenylpropanol; 1-Ethyl-1-methyl-3-phenylpropanol; 2-Methyl-5-phenylpentanol; 3-Methyl-5-phenylpentanol; 3-Phenyl-2-propen-1-ol; 4-Methoxybenzylalkohol; 1-(4-Isopropylphenyl)ethanol;
der Ester von araliphatischen Alkoholen und aliphatischen Carbonsäuren wie z.B. Benzylacetat; Benzylpropionat; Benzylisobutyrat; Benzylisovalerianat; 2-Phenylethylacetat; 2-Phenylethylpropionat; 2-Phenylethylisobutyrat; 2-Phenylethylisovalerianat; 1-Phenylethylacetat; alpha-Trichlormethylbenzylacetat; alpha,alpha-Dimethylphenylethylacetat; alpha,alpha-Dimethylphenylethylbutyrat; Cinnamylacetat; 2-Phenoxyethylisobutyrat; 4-Methoxybenzylacetat;
der araliphatischen Ether wie z.B. 2-Phenylethylmethylether; 2-Phenylethylisoamylether; 2-Phenylethyl-1-ethoxyethylether; Phenylacetaldehyddimethylacetal; Phenylacetaldehyddiethylacetal; Hydratropaaldehyddimethylacetal; Phenylacetaldehydglycerinacetal; 2,4,6-Trimethyl-4-phenyl-1,3-dioxan; 4,4a,5,9b-Tetrahydroindeno[1,2-d]-m-dioxin; 4,4a,5,9b-Tetrahydro-2,4-dimethylindeno[1,2-d]-m-dioxin;
der aromatischen und araliphatischen Aldehyde wie z.B. Benzaldehyd; Phenylacetaldehyd; 3-Phenylpropanal; Hydratropaaldehyd; 4-Methylbenzaldehyd; 4-Methylphenylacetaldehyd; 3-(4-Ethylphenyl)-2,2-dimethylpropanal; 2-Methyl-3-(4-isopropylphenyl)propanal; 2-Methyl-3-(4-tert.-butylphenyl)propanal; 2-Methyl-3-(4-isobutylphenyl)propanal; 3-(4-tert.-Butylphenyl)propanal; Zimtaldehyd; alpha-Butylzimtaldehyd; alpha-Amylzimtaldehyd; alpha-Hexylzimtaldehyd; 3-Methyl-5-phenylpentanal; 4-Methoxybenzaldehyd; 4-Hydroxy-3-methoxybenzaldehyd; 4-Hydroxy-3-ethoxybenzaldehyd; 3,4-Methylendioxybenzaldehyd; 3,4-Dimethoxybenzaldehyd; 2-Methyl-3-(4-methoxyphenyl)propanal; 2-Methyl-3-(4-methylendioxyphenyl)propanal;
der aromatischen und araliphatischen Ketone wie z.B. Acetophenon; 4-Methylacetophenon; 4-Methoxyacetophenon; 4-tert.-Butyl-2,6-dimethylacetophenon; 4-Phenyl-2-butanon; 4-(4-Hydroxyphenyl)-2-butanon; 1-(2-Naphthalenyl)ethanon;2-Benzofuranylethanon;(3-Methyl-2-benzofuranyl)ethanon; Benzophenon; 1,1,2,3,3,6-Hexamethyl-5-indanylmethylketon; 6-tert.-Butyl-1,1-dimethyl-4-indanylmethylketon; 1-[2,3-dihydro-1,1,2,6-tetramethyl-3-(1-methylethyl)-1H-5-indenyl]ethanon; 5',6',7',8'-Tetrahydro-3',5',5',6',8',8'-hexamethyl-2-acetonaphthon;
der aromatischen und araliphatischen Carbonsäuren und deren Ester wie z.B. Benzoesäure; Phenylessigsäure; Methylbenzoat; Ethylbenzoat; Hexylbenzoat; Benzylbenzoat; Methylphenylacetat; Ethylphenylacetat; Geranylphenylacetat; Phenylethylphenylacetat; Methylcinnmat; Ethylcinnamat; Benzylcinnamat; Phenylethylcinnamat; Cinnamylcinnamat; Allylphenoxyacetat; Methylsalicylat; Isoamylsalicylat; Hexylsalicylat; Cyclohexylsalicylat; Cis-3-Hexenylsalicylat; Benzylsalicylat; Phenylethylsalicylat; Methyl-2,4-dihydroxy-3,6-dimethylbenzoat; Ethyl-3-phenylglycidat; Ethyl-3-methyl-3-phenylglycidat;
der stickstoffhaltigen aromatischen Verbindungen wie z.B. 2,4,6-Trinitro-1,3-dimethyl-5-tert.-butylbenzol; 3,5-Dinitro-2,6-dimethyl-4-tert.-butylacetophenon; Zimtsäurenitril; 3-Methyl-5-phenyl-2-pentensäurenitril; 3-Methyl-5-phenylpentansäurenitril; Methylanthranilat; Methy-N-methylanthranilat; Schiff'sche Basen von Methylanthranilat mit 7-Hydroxy-3,7-dimethyloctanal, 2-Methyl-3-(4-tert.-butylphenyl)propanal oder 2,4-Dimethyl-3-cyclohexencarbaldehyd; 6-Isopropylchinolin; 6-Isobutylchinolin; 6-sec.-Butylchinolin;2-(3-Phenylpropyl)pyridin; Indol; Skatol; 2-Methoxy-3-isopropylpyrazin; 2-Isobutyl-3-methoxypyrazin;
der Phenole, Phenylether und Phenylester wie z.B. Estragol; Anethol; Eugenol; Eugenylmethylether; Isoeugenol; Isoeugenylmethylether; Thymol; Carvacrol; Diphenylether; beta-Naphthylmethylether; beta-Naphthylethylether; beta-Naphthylisobutylether; 1,4-Dimethoxybenzol; Eugenylacetat; 2-Methoxy-4-methylphenol; 2-Ethoxy-5-(1-propenyl)phenol; p-Kresylphenylacetat;
der heterocyclischen Verbindungen wie z.B. 2,5-Dimethyl-4-hydroxy-2H-furan-3-on; 2-Ethyl-4-hydroxy-5-methyl-2H-furan-3-on; 3-Hydroxy-2-methyl-4H-pyran-4-on; 2-Ethyl-3-hydroxy-4H-pyran-4-on;
der Lactone wie z.B. 1,4-Octanolid; 3-Methyl-1,4-octanolid; 1,4-Nonanolid; 1,4-Decanolid; 8-Decen-1,4-olid; 1,4-Undecanolid; 1,4-Dodecanolid; 1,5-Decanolid; 1,5-Dodecanolid; 4-Methyl-1,4-decanolid; 1,15-Pentadecanolid; cis- und trans-11-Pentadecen-1,15-olid; cis- und trans-12-Pentadecen-1,15-olid; 1,16-Hexadecanolid; 9-Hexadecen-1,16-olid; 10-Oxa-1,16-hexadecanolid; 11-Oxa-1,16-hexadecanolid; 12-Oxa-1,16-hexadecanolid; Ethylen-1,12-dodecandioat; Ethylen-1,13-tridecandioat; Cumarin; 2,3-Dihydrocumarin; Octahydrocumarin.

Wie bereits mehrfach erwähnt, eignet sich 2,4'-Dimethyl-propiophenon wegen seiner olfaktorischen Eigenschaften besonders gut für den Einsatz in Riechstoffkompositionen. Die Verbindung kann dabei in entsprechenden Riechstoffkompositionen in Verbindung mit einem weiteren Einzelstoff oder aber einer Vielzahl weiterer Riechstoffe kombiniert eingesetzt werden. Besonders vorteilhaft lässt sich die Verbindung mit anderen Riechstoffen , vorzugsweise ausgewählt aus den bereits oben bzw. weiter unten genannten Riechstoffen, in verschiedenen, unterschiedlichen Mengenverhältnissen zu neuartigen Riechstoff- bzw. Parfümkompositionen kombinieren.

Zuvor wurde bereits ausgeführt, dass sich 2,4'-Dimethyl-propiophenon erfindungsgemäß besonders gut zur Verwendung als Bestandteil in Riechstoffkompositionen des Fougére-Typs oder des Rosen -Typs eignet. Demnach betrifft ein weiterer Aspekt der vorliegenden Erfindung eine Riechstoffkomposition (wie oben definiert) mit einer Rosennote, welche zumindest einen weiteren Rosenriechstoff, vorzugsweise 2, 3, 4 oder mehr weitere Rosenriechstoffe, enthält, wobei der oder die weiteren Rosenriechstoffe ausgewählt sind aus der Gruppe bestehend aus:
Geranylformiat; Geranylacetat; Geranylpropionat; Geranylisobutyrat; Geranylbutyrat; Geranylisovalerianat; Geranyltiglinat; Geranylbenzoat; Citronellylformiat; Citronellylacetat; Citronellylpropionat; Citronellylisobutyrat; Citronellylbutyrat; Citronellylisovalerianat; Citronellyltiglinat; Citronellylbenzoat; Phenylethylalkohol; Phenylacetaldehyd; Phenylacetaaldehyddimethylacetal; Phenylethylformiat; Phenylethylacetat; Phenylethylpropionat; Phenylethylisobutyrat; Phenylethylbutyrat; Phenylethylpivalat; Phenylethylisovalerianat; Phenylethyl-2-ethylbutyrat; Phenylethyltiglinat; Phenylethylbenzoat; Phenylethylphenylacetat; 2-Phenoxyethylisobutyrat; Geranylmethylether; Rosenoxid; Phenylethylmethylether; Phenylethylethylether; Phenylethylisoamylether; 2-Methoxybenzylethylether; alpha-Trichlormethylbenzylacetat; alpha,3,3-Trimethylcyclohexanmethylacetat; 2,4,6-Trimethyl-3-cyclohexenmethanol; N,N-Diethyl-2-ethylhexansäureamid; 3,7-Dimethyloctanol; Geranylaceton; Linalool; 3,7-Dimethyl-1,6-nonadien-3-ol; Nerolidol; Farnesol; 9-Decenol; 9-Decenylacetat; Decanal; 10-Undecenal; 10-Undecenol; Citronellyloxyacetaldehyd; 3,7-Dimethyloctanylacetaldehyd; 2-Methyl-5-phenylpentanol; 2-Methyl-5-phenylpentanal; 3-Methyl-5-phenylpentanol; Benzophenon; Diphenyloxid; Diphenylmethan; alpha -Damascon; beta-Damascon; delta-Damascon; gamma-Damascon; beta-Damascenon; 1-(2,4,4-Trimethyl-2-cyclohexenyl)-2-buten-1-on; Rosenöl; Rosenabsolue; Geraniumöl, Geraniol, Nerol, 2-Phenylethylalkohol.

Ganz besonders bevorzugt ist jedoch ein entsprechender Einsatz von 2,4'-Dimethyl-propiophenon in Fougére-Duftthemen. Eine ganz besonders bevorzugte Ausführungsform einer erfindungsgemäßen Riechstoffkomposition ist demnach eine Riechstoffkomposition mit einer Fougére-Note, welche zumindest einen weiteren herbal-krautigen Riechstoff, vorzugsweise 2, 3, 4 oder mehr weitere herbal-krautige Riechstoffe enthält , wobei der eine oder die mehreren weiteren herbal-krautigen Riechstoffe ausgewählt sind aus der Gruppe bestehend aus:
4,7-Methano-3a,4,5,6,7,7a-hexahydro-5 (oder -6) -indenylacetate; eta-Orcincarbonsäure-methylester; Terpinylacetat; Terpineol; Terpinenol-4; Linalool; Campher; 1,8-Cineol; Lavandulol; Lavandulylacetat; 1-Octen-3-ol; Caryophyllen; Farnesen; 2-Butyl-4,4,6-trimethyl-1,3-dioxan.

Wie bereits erwähnt, werden die besonderen positiven Eigenschaften von 2,4'-Dimethyl-propiophenon im Zusammenhang mit Fougére-Duftthemen bzw. Riechstoffkompositionen mit einer Fougére-Note, weiter unten im Rahmen der erfindungsgemäßen Verwendungen sowie anhand des Fougére-Duftthemabeispiels (Beispiel 2) weiter erläutert.

Erfindungsgemäße Riechstoffkompositionen bzw. das erfindungsgemäß zu verwendende 2,4'-Dimethyl-propiophenon werden vorzugsweise zur Herstellung parfümierter Artikel eingesetzt. Die sensorischen Eigenschaften ebenso wie die stofflichen Eigenschaften (wie Löslichkeit in gängigen Lösungsmitteln und Kompatibilität mit gängigen weiteren Bestandteilen derartiger Produkte) sowie die toxikologische Unbedenklichkeit der erfindungsgemäß einzusetzenden Verbind ung unterstreichen ihre besondere Eignung für die genannten Einsatzzwecke. Die weiter unten im Zusammenhang mit weiteren erfindungsgemäßen Verwendungen beschriebenen positiven Eigenschaften tragen dazu bei, dass die erfindungsgemäßen Riechstoffkompositionen besonders bevorzugt in Waschmitteln, Hygiene- oder Pflegeprodukten, insbesondere im Bereich der Körper- und Haarpflege, der Kosmetik und des Haushalts eingesetzt werden.

Dementsprechend wird im Rahmen der vorliegenden Erfindung ein parfümierter Artikel angegeben, welcher eine wie oben definierte erfindungsgemäße Riechstoffkomposition enthält. Bevorzugte erfindungsgemäße Artikel sind ausgewählt aus der Gruppe bestehend aus:
Parfüm-Extraits, Eau de Parfums, Eau de Toilettes, Rasierwässer, Eau de Colognes, Pre-shave-Produkte, Splash-Colognes, parfümierte Erfrischungstücher, Parfüms für saure, alkalische und neutrale Reinigungsmittel, Waschmittel, Waschtabletten, Desinfektionsmitteln, sowie von Luftverbesserern, Aerosolsprays, Wachse und Polituren, sowie Körperpflegemitteln, Badeölen, kosmetischen Emulsionen, wie z.B. Hautcremes- und -lotionen, Sonnenschutzcremes- und -lotionen, After-sun-cremes und -lotionen, Handcremes und -lotionen, Fußcremes und -lotionen, Enthaarungscremes und -lotionen, After-shave-Cremes und -lotionen, Bräunungscremes und -lotionen, Haarpflegeprodukten wie z.B. Haarsprays, Haargelen, festigende Haarlotionen, Haarspülungen, Haarfärbemitteln, Haarverformungsmitteln und Haarglättungsmitteln, Haarwässern, Haarcremes und -lotionen, Deodorantien und Antiperspirantiens, Produkten der dekorativen Kosmetik wie z.B. Lidschatten, Nagellacke, Make-ups, Lippenstifte, Mascara sowie von Kerzen, Lampenölen, Räucherstäbchen, Insektiziden, Repellentien, Treibstoffen.

Erfindungsgemäße Riechstoffkompositionen enthaltend 2,4'-Dimethyl-propiophenon können aber allgemein (z.B. in konzentrierter Form, in Lösungen oder in unten beschriebener modifizierter Form) verwendet werden für die Herstellung von z.B. Parfüm-Extraits, Eau de Parfums, Eau de Toilettes, Rasierwässer, Eau de Colognes, Pre-shave-Produkte, Splash-Colognes und parfümierten Erfrischungstüchern sowie die Parfümierung von sauren, alkalischen und neutralen Reinigungsmitteln, wie z.B. Fußbodenreinigern, Fensterglasreinigern, Geschirrspülmittel, Bad- und Sanitärreinigern, Scheuermilch, festen und flüssigen WC-Reinigern, pulver- und schaumförmigen Teppichreinigern, flüssigen Waschmitteln, pulverförmigen Waschmitteln, Wäschevorbehandlungsmitteln wie Bleichmittel, Einweichmittel und Fleckenentfernern, Wäscheweichspülern, Waschseifen, Waschtabletten, Desinfektionsmitteln, Oberflächendesinfektionsmitteln sowie von Luftverbesserern in flüssiger, gelartiger oder auf einem festen Träger aufgebrachter Form, Aerosolsprays, Wachsen und Polituren wie Möbelpolituren, Fußbodenwachsen, Schuhcremes sowie Körperpflegemitteln wie z.B. festen und flüssigen Seifen, Duschgelen, Shampoos, Rasierseifen, Rasierschäumen, Badeölen, kosmetischen Emulsionen vom Öl-in-Wasser-, vom Wasser-in-Öl- und vom Wasser-in-Öl-in-Wasser-Typ wie z.B. Hautcremes- und -lotionen, Gesichtscremes und -lotionen, Sonnenschutzcremes-und -lotionen, After-sun-cremes und -lotionen, Handcremes und -lotionen, Fußcremes und -lotionen, Enthaarungscremes und -lotionen, After-shave-Cremes und -lotionen, Bräunungscremes und -lotionen, Haarpflegeprodukten wie z.B. Haarsprays, Haargelen, festigen Haarlotionen, Haarspülungen, permanenten und semipermanenten Haarfärbemitteln, Haarverformungsmitteln wie Kaltwellen und Haarglättungsmitteln, Haarwässern, Haarcremes und -lotionen, Deodorantien und Antiperspirantien wie z.B. Achselsprays, Roll-ons, Deosticks, Deocremes, Produkten der dekorativen Kosmetik wie z.B. Lidschatten, Nagellacke, Make-ups, Lippenstifte, Mascara sowie von Kerzen, Lampenölen, Räucherstäbchen, Insektiziden, Repellentien, Treibstoffen.

Für die in erfindungsgemäßen parfümierten Artikeln enthaltenen Riechstoffkomposition und deren bevorzugte Ausführungsformen gilt das weiter oben Gesagte entsprechend. Die erfindungsgemäßen zuvor genannten Riechstoffkompositionen bzw. die erfindungsgemäß in den entsprechenden Artikeln einzusetzenden Riechstoffkompositionen können in flüssiger Form, unverdünnt oder mit einem Lösungmittel verdünnt für Parfümierungen eingesetzt werden. Geeignete Lösungsmittel hierfür sind z.B. Ethanol, Isopropanol, Diethylenglycolmonoethylether, Glycerin, Propylenglycol, 1,2-Butylenglycol, Dipropylenglycol, Diethylphthalat, Triethylcitrat, Isopropylmyristat usw. Für die genannten Lösungsmittel gilt, dass diese, im Rahmen des vorliegenden Textes, im Falle des Vorhandenseins eigener olfaktorischer Eigenschaften ausschließlich dem Bestandteil "Lösungsmittel" und nicht den "Riechstoffen" zuzuordnen sind.

Die in den erfindungsgemäßen parfümierten Artikeln enthaltenen Riechstoffkompositionen können dabei in einer bevorzugten Ausführungsform an einem Trägerstoff absorbiert sein, der sowohl für eine feine Verteilung der Riechstoffe im Produkt als auch für eine kontrollierte Freisetzung bei der Anwendung sorgt. Derartige Träger können poröse anorganische Materialien wie Leichtsulfat, Kieselgele, Zeolithe, Gipse, Tone, Tongranulate, Gasbeton usw. oder organische Materialien wie Hölzer und Cellulose-basierende Stoffe sein.

Die erfindungsgemäßen Riechstoffkompostionen können auch mikroverkapselt, sprühgetrocknet, als Einschluss-Komplexe oder als Extrusions-Produkte vorliegen und in dieser Form dem zu parfümierenden Produkt, bzw. Artikel, hinzugefügt werden.

Gegebenenfalls können die Eigenschaften der derart modifizierten Riechstoffkompositionen durch sogenanntes "Coaten" mit geeigneten Materialien im Hinblick auf eine gezieltere Duftfreisetzung weiter optimiert werden, wozu vorzugsweise wachsartige Kunststoffe wie z.B. Polyvinylalkohol verwendet werden.

Die Mikroverkapselung der Riechstoffkompositionen kann beispielsweise durch das sogenannte Koazervationsverfahren mit Hilfe von Kapselmaterialien, z.B. aus polyurethan-artigen Stoffen oder Weichgelatine, erfolgen. Die sprühgetrockneten Parfümöle können beispielsweise durch Sprühtrocknung einer das Parfümöl enthaltenden Emulsion bzw. Dispersion hergestellt werden, wobei als Trägerstoffe modifizierte Stärken, Proteine, Dextrin und pflanzliche Gummen verwendet werden können. Einschluss-Komplexe können z.B. durch Eintragen von Dispersionen von der Riechstoffkomposition und Cyclodextrinen oder Harnstoffderivaten in ein geeignetes Lösungsmittel, z.B. Wasser, hergestellt werden. Extrusions-Produkte können durch Verschmelzen der Riechstoffkomposition mit einem geeigneten wachsartigen Stoff und durch Extrusion mit nachfolgender Erstarrung, ggf. in einem geeigneten Lösungsmittel, z.B. Isopropanol, erfolgen.

Die erfindungsgemäßen Riechstoffkompositionen können demnach, wie bereits erwähnt, in konzentrierter Form, in Lösungen oder in oben beschriebener modifizierter Form für die Herstellung der entsprechenden erfindungsgemäßen parfümierten Artikel verwendet werden

Zutaten, mit denen 2,4'-Dimethyl-propiophenon vorzugsweise kombiniert werden kann, sind beispielsweise: Konservierungsmittel, Abrasiva, Antiakne-Mittel, Mittel gegen Hautalterung, antibakterielle Mittel, Anticellulitis-Mittel, Antischuppen-Mittel, entzündungshemmende Mittel, irritationsverhindernde Mittel, irritationshemmende Mittel, antimikrobielle Mittel, Antioxidantien, Adstringentien, schweisshemmende Mittel, antiseptische Mittel, Antistatika, Binder, Puffer, Trägermaterialien, Chelatbildnder, Zellstimulantien, reinigende Mittel, pflegende Mittel, Enthaarungsmittel, oberflächenaktive Substanzen, deodorierende Mittel, Antiperspirantien, Weichmacher, Emulgatoren, Enzyme, ätherische Öle, Fasern, Filmbildner, Fixateure, Schaumbildner, Schaumstabilisatoren, Substanzen zum Verhindern des Schäumens, Schaumbooster, Fungizide, gelierende Mittel, gelbildende Mittel, Haarpflegemittel, Haarverformungsmittel, Haarglättungsmittel, feuchtigkeitsspendende Mittel, anfeuchtende Substanzen, feuchthaltende Substanzen, bleichende Mittel, stärkende Mittel, fleckenentfernende Mittel, optisch aufhellende Mittel, imprägnierende Mittel, schmutzabweisende Mittel, reibungsverringernde Mittel, Gleitmittel, Feuchtigkeitscremes, Salben, Trübungsmittel, plastifizierende Mittel, deckfähige Mittel, Politur, Glanzmittel, Polymere, Pulver, Proteine, rückfettende Mittel, abschleifende Mittel, Silicone, hautberuhigende Mittel, hautreinigende Mittel, hautpflegende Mittel, hautheilende Mittel, Hautaufhellungsmittel, hautschützende Mittel, hauterweichende Mittel, kühlende Mittel, hautkühlende Mittel, wärmende Mittel, hautwärmende Mittel, Stabilisatoren, UV-absorbierende Mittel, UV-Filter, Waschmittel, Weichspüler, suspendierende Mittel, Hautbräunungsmittel, Verdickungsmittel, Vitamine, Öle, Wachse, Fette, Phospholipide, gesättigte Fettsäuren, ein- oder mehrfach ungesättigte Fettsäuren, α-Hydroxysäuren, Polyhydroxyfettsäuren, Verflüssiger, Farbstoffe, farbschützende Mittel, Pigmente, Antikorrosiva, Aromen, Geschmackstoffe, Riechstoffe, Polyole, Tenside, Elektrolyte, organische Lösungsmittel oder Silikonderivate.

Im Folgenden werden weitere erfindungsgemäße Verwendungen von 2,4'-Dimethyl-propiophenon bzw. 2,4'-Dimethyl-propiophenon-enthaltenden Mischungen beschrieben.

Für tensidhaltige Produkte ist die Substantivität der Riechstoffe bzw. der Riechstoffkompositionen gegenüber den bzw. deren Retention am Substrat, insbesondere Haaren oder textilen Fasern, eine weitere wichtige anwendungstechnische Anforderung an die erfindungsgemäße Riechstoffmischung. Überraschenderweise zeigt sich, dass sich 2,4'-Dimethyl-propiophenon besonders, insbesondere für einen blumigen Riechstoff, durch ein hohes Aufziehvermögen (Eigenhaftung auf einem Substrat) und eine hohe Substantivität (Fähigkeit aus einer, meist wässrigen, Phase heraus auf ein Substrat aufzuziehen bzw. auch nach einem Wasch- oder Spülvorgang auf einem Substrat zu verbleiben) auszeichnet. Dieser Effekt wird weiter unten im Rahmen eines Beispiels noch näher erläutert.

Dementsprechend betrifft ein weiterer Aspekt der vorliegenden Erfindung die Verwendung von 2,4'-Dimethyl-propiophenon als Mittel zum Erhöhen der Substantivität und/oder Retention einer Riechstoffkomposition.

Ferner kann 2,4'-Dimethyl-propiophenon oder eine 2,4'-Dimethyl-propiophenon enthaltende Mischung als Mittel verwendet werden zum Versehen von (a) Haaren oder (b) textilen Fasern mit einer, zwei, drei oder sämtlichen der Geruchsnoten blumig, rosig, rosenoxidartig und herbalartig, vorzugsweise mit einer rosenoxidartigen und herbalartigen Geruchsnote.

Das erfindungsgemäß zu verwendende 2,4'-Dimethyl-propiophenon eignet sich daher besonders gut für den Einsatz in tensidhaltigen Produkten.

Bevorzugt ist es, wenn das Produkt eines der folgenden ist:
- ein saures, alkalisches oder neutrales Reinigungsmittel, das insbesondere aus der Gruppe ausgewählt ist bestehend aus Allzweckreinigern, Fußbodenreinigern, Fensterglasreinigern, Geschirrspülmittel, Bad- und Sanitärreinigern, Scheuermilch, festen und flüssigen WC-Reinigern, pulver- und schaumförmigen Teppichreinigern, flüssigen Waschmitteln, pulverförmigen Waschmitteln, Wäschevorbehandlungsmitteln wie Bleichmittel, Einweichmittel und Fleckenentfernern, Wäscheweichspülern, Waschseifen, Waschtabletten, Desinfektionsmitteln, Oberflächendesinfektionsmitteln,
- ein Luftverbesserer in flüssiger, gelartiger oder auf einem festen Träger aufgebrachter Form oder als Aerosolspray,
- ein Wachs oder eine Politur, die insbesondere aus der Gruppe ausgewählt ist bestehend aus Möbelpolituren, Fußbodenwachsen und Schuhcremes, oder
- ein Körperpflegemittel, das insbesondere aus der Gruppe ausgewählt ist bestehend aus Duschgelen und Shampoos.

Gemäß einem weiteren Aspekt der vorliegenden Erfindung wird die Verwendung von 2,4'-Dimethyl-propiophenon als Mittel zum Erhöhen der Komplexstabilität einer Riechstoffkomposition angegeben.

Überraschenderweise zeigte sich in eigenen Untersuchungen, dass 2,4'-Dimethyl-propiophenon in den erfindungsgemäßen Riechstoffkompositionen, vorzugsweise in einer der vorstehend als bevorzugt angegebenen Ausgestaltungen, - figürlichen ausgedrückt - ein "Zusammenrücken" der Einzelbestandteile der Komposition bewirkt, d.h. die Komplexstabilität verbessert bzw. verlängert. Das bedeutet, dass z.B. das Abdampfverhalten der einzelnen Riechstoffe bzw. der Riechstoffkomponenten linearisiert und olfaktorisch synchronisiert wird. Dieser Effekt zeigt sich vor allem in den bevorzugten Riechstoffkompositionen des Fougére-Typs.

Zudem wird eine Verwendung von 2,4'-Dimethyl-propiophenon als Mittel zum Verändern, Maskieren und/oder Vermindern einer medizinischen Geruchsnote in einer Riechstoffkomposition angegeben, vorzugsweise in einer erfindungsgemäßen Riechstoffkomposition. In eigenen Untersuchungen zeigte sich erneut am Beispiel einer Riechstoffkomposition des Fougére-Typs, dass 2,4'-Dimethyl-propiophenon der Riechstoffkomposition bzw. den darin enthaltenen herbalen Kräutemoten den medizinischen Duftcharakter nimmt bzw. ihn zumindest reduziert. Dadurch fördert 2,4'-Dimethyl-propiophenon die Balance der Riechstoffkomposition und erzeugt ein parfümistisch reiferes Erscheinungsbild. Eine erfindungsgemäße Verwendung in einer Riehstoffkomposition des Fougére-Typs ist daher besonders bevorzugt.

Gesucht werden häufig Riechstoffmischungen mit einer rosenoxid- und herbalartigen Kopfnote, wobei diese gleichzeitig ein ausgeprägtes Blooming (Geruch aus einer wässrigen Lösung) aufweisen sollten. Wie im Rahmen der Ausführungsbeispiele weiter unten gezeigt wird, eignet sich das erfindungsgemäß zu verwendende 2,4'-Dimethyl-propiophenon auch für diesen Einsatzzweck.

Ein weiterer Aspekt der vorliegenden Erfindung betrifft demnach die Verwendung von 2,4'-Dimethyl-propiophenon als Mittel zum Erhöhen des über einer tensidhaltigen wässrigen Lösung wahrgenommenen Geruches anderer Riechstoffe.

Das erfindungsgemäß als Riechstoff in Riechstoffkompositionen zu verwendende 2,4'-Dimethyl-propiophenon ist durch eine in gängigen präparativen Standardwerken beschriebene Friedel-Crafts-Acylierung herstellbar. In einem bevorzugten Herstellverfahren wird Toluol im Überschuss eingesetzt und dient somit sowohl als Reagenz als auch als Lösungsmittel. Die bevorzugte Lewissäure Aluminiumchlorid wird vorzugsweise vorgelegt und die Reaktionstemperatur durch die Dosiergeschwindigkeit des Isobuttersäurehalogenids, vorzugsweise des Isobuttersäurechlorids, kontrolliert. Als Nebenprodukte entstehen die regioisomeren meta-(1,7%) und ortho-(3,8%) Verbindungen.

Die Reaktion kann anhand des folgenden Schemas beispielhaft veranschaulicht werden:

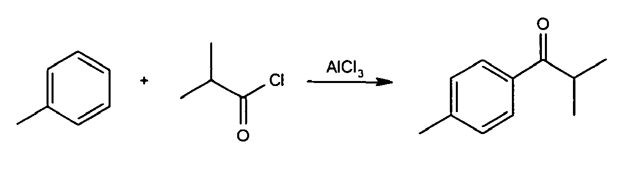

Das heißt, je nach Acylierungsbedingungen kann das Reaktionsprodukt auch die isomeren orto- und meta-Ketone enthalten. Dementsprechend kann auch eine erfindungsgemäße Riechstoffmischung bzw. ein erfindungsgemäßer parfümierter Artikel die isomeren orto- und meta-Ketone enthalten.

Eine erfindungsgemäße Riechstoffkomposition wird erfindungsgemäß hergestellt, indem 2,4'-Dimethyl-propiophenon mit dem oder den weiteren Riechstoffen sowie gegebenenfalls weiteren Bestandteilen der Riechstoffkomposition vermischt wird.

Gemäß einer bevorzugten Ausführungsform wird eine erfindungsgemäße Riechstoffkomposition wie oben beschrieben hergestellt, wobei jedoch das 2,4'-Dimethyl-propiophenon in einer Menge eingesetzt wird, die ausreicht, um in der Riechstoffkomposition eine, zwei, drei oder sämtliche der Geruchsnoten blumig, rosig, rosenoxidartig und herbalartig, vorzugsweise beide der Geruchsnoten rosenoxidartig und herbalartig, zu vermitteln, zu modifizieren und/oder zu verstärken.

Für die neben 2,4'-Dimethyl-propiophenon bevorzugt auszuwählenden weiteren Bestandteile bzw. Riechstoffe gilt das weiter oben Gesagte entsprechend.

Wie bereits erwähnt kann 2,4'-Dimethyl-propiophenon erfindungsgemäß bevorzugt zur Verwendung als Mittel zum Versehen von Haaren oder textilen Fasern einer entsprechenden erwünschten Geruchsnote verwendet werden.

Dementsprechend betrifft ein weiterer Aspekt der vorliegenden Erfindung ein Verfahren zum Versehen von (a) Haaren oder (b) textilen Fasern mit einer, zwei, drei oder sämtlichen der Geruchsnoten blumig, rosig, rosenoxidartig und herbalartig, vorzugsweise mit einer rosenoxidartigen und herbalartigen Geruchsnote, umfassend die folgenden Schritte:
i) Bereitstellen einer Mischung, umfassend 2,4'-Dimethyl-propiophenon oder eine erfindungsgemäße Riechstoffkomposition wie weiter oben definiert,
ii) Applizieren der Mischung auf (a) das Haar oder (b) die textilen Fasern.

Nachfolgend wird die Erfindung anhand von Beispielen näher erläutert.

### Beispiel 1: Darstellung von 2,4'-Dimethyl-propiophenon

368 g Toluol (4 mol) und 200 g (1,50 mol) AlCl₃ werden bei 0°C vorgelegt. Dann werden 159 g (1,49 mol) Isobuttersäurechlorid bei 0°C innerhalb von 2 Stunden schrittweise zudosiert. Nach Dosierende wird 30 Minuten bei einer Temperatur von 0°C bis 8°C nachgerührt. Der Reaktionsansatz wird auf 318 g Wasser / 440 g Eis gegeben. Die wässrige Phase wird abgetrennt, die organische Phase zweimal mit je 160 g Wasser nachgewaschen. Es werden 458 g 2,4'-Dimethyl-propiophenon roh in Toluol erhalten, die mit ca. 1 g gesättigter Natriumbicarbonat-Lösung auf einen pH-Wert von 8,5 eingestellt werden. Nach Abdestillation von Toluol und Wasser destilliert das Produkt bei einer Kopftemperatur von 85 bis 88°C und einem Vakuum von 2 mbar über. Es werden 225 g 2,4'-Dimethyl-propiophenon Destillat erhalten.

Die Isomerenverteilung im gequenchten Reaktionsansatz ist gemäß der GC-Auswertung (Kapillarsäule DB-1, Kaltinjektion) wie folgt:
2,4'-Dimethyl-propiophenon (para) 94,4%, 2,3'-Dimethyl-propiophenon (meta) 1,7% (Geruch: schwächer als para und ortho, rosig, technisch) und 2,2'-Dimethyl-propiophenon (ortho) 3,8% (Geruch: stärker als meta, rosig, pudrig, staubig).

Die 2,4'-Dimethyl-propiophenon Ausbeute d. Th. bezogen auf Isobuttersäurechlorid liegt bei 87,9%.

Spektroskopische und spektrometrische Daten von 2,4'-Dimethyl-propiophenon:
¹H-NMR (CDCl₃, 400 MHz, TMS= 0 ppm): δ = 1,21 ppm (d, 2 -CH₃, 6 H), 2,41 (s, -CH₃, 3 H), 3,54 (sept, -CH, 1 H), 7,26 (m, 2 -CH, 2 H), 7,86 (m, 2 -CH, 2 H).
¹³C-NMR (CDCl₃, 75 MHz): δ = 19,22 ppm (2 C), 21,56 , 35,20 , 128,46 (2 C), 129,29 (2 C), 133,70 , 143,51 , 204,16 .
MS (m/e, %): 162 (M, 8 %), 120 (10), 119 (100), 91 (33), 65 (12), 39 (5).

### Beispiel 2: Riechstoffkomposition mit und ohne 2,4'-Dimethyl-propiophenon

| | Parfümöl AE | Parfümöl AB |
|---|---|---|
| n-Dodecanal | 3 | 3 |
| 2-Methylundecanal | 7 | 7 |
| Vertocitral¹⁾ | 10 | 10 |
| Galbanum Resin | 3 | 3 |
| Styrolylacetat | 7 | 7 |
| Terpinylacetat | 30 | 30 |
| Citral | 10 | 10 |
| Orangenöl, italienisch | 20 | 20 |
| Claritone^{®1)} | 10 | 10 |
| Petitgrainöl | 20 | 20 |
| Lavandinöl Grosso | 100 | 100 |
| Spiköl, spanisch | 100 | 100 |
| Rosmarinöl | 60 | 60 |
| Salbeiöl, spanisch | 30 | 30 |
| Dillkrautöl | 10 | 10 |
| Beifußöl | 10 | 10 |
| Lyral^{®2)} | 50 | 50 |
| Hedione³⁾ | 50 | 50 |
| Methyloctincarbonat | 1 | 1 |
| Herbaflorat¹⁾ | 25 | 25 |
| Herbylpropionat | 20 | 20 |
| Iso E Super²⁾ | 70 | 70 |
| Vetiveröl Java | 10 | 10 |
| Vetikolacetat^{®1)} | 5 | 5 |
| Patchouliöl | 30 | 30 |
| Evernyl⁴⁾ | 5 | 5 |
| Ambroxid krist.¹⁾ | 4 | 4 |
| 2,4'-Dimethyl-propiophenon | 70 | - |
| DPG (Dipropylenglycol) | 230 | 300 |
| | 1000 | 1000 |

| | | |
|---|---|---|
| ¹⁾ Handelsname der Fa. Symrise, Holzminden, D ²⁾ Handelsname der Fa. IFF, New Jersey, US ³⁾ Handelsname der Fa. Firmenich, Genf, CH ⁴⁾ Handelsname der Fa. Givaudan, Zürich, CH | | |

Bei den Demonstrationsbeispielen der Parfümöle AE/AB handelt es sich um Riechstoffkompositionen, im Speziellen um parfümistische Kompositionen des Fougére-Typs, bestehend aus natürlichen, synthetisch-naturidentischen und vollsynthetischen Riechstoffen.

Das Profil des Duftes ist so gewählt, dass Stoffe mit einem Blattgrün-Geruch, nach Citrusfrüchten riechende Komponenten, herbig-krautig-würzig und medizinisch riechende Kräuteröle und Materialien mit trocken-holzigen Anklängen miteinander in einen duftgemäßen Zusammenklang treten.

Durch unterschiedliche chemisch-physikalische Eigenschaften der Einzelkomponenten bedingt, zeigt der Dufteindruck nicht in allen Phasen der Evaporation die erwünschte Komplexität auf.

Wie im Rahmen der Beschreibung der vorliegenden Erfindung bereits erwähnt, bietet der Einsatz von 2,4'-Dimethyl-propiophenon als Riechstoff zahlreiche Vorteile. 2,4'-Dimethyl-propiophenon bewirkt auch in der gewählten Konzentration des Ausführungsbeispiel ein (im figürlichen Sinne) "Zusammenrücken" der Einzelbestandteile. Das bedeutet, das Abdampfverhalten der einzelnen Komponenten wird linearisiert und olfaktorisch synchronisiert. Dadurch wird die Komplexstabilität der Riechstoffkomposition verlängert.

Die herbale Note des Geruchsprofils von 2,4'-Dimethyl-propiophenon zeichnet sich mehr durch einen würzigen, grünen, in Richtung krautigen Charakter aus und ist weniger als medizinisch anzusehen. In der Riechstoffkomposition werden die würzigen und grünen, krautigen Aspekte durch 2,4'-Dimethyl-propiophenon gestärkt und die medizinischen vermindert. 2,4'-Dimethyl-propiophenon wirkt somit harmonisierend auf die herbalen Kräuternoten und nimmt ihnen bzw. reduziert den medizinischen Duftcharakter. 2,4'-Dimethyl-propiophenon fördert die Balance der Riechstoffkomposition und erzeugt ein parfümistisch reiferes Erscheinungsbild.

Die weiteren im Vergleich zu bisherigen Marktprodukten besseren anwendungstechnischen Eigenschaften von 2,4'-Dimethyl-propiophenon werden nachfolgend an einigen Beispielen verdeutlicht.

### Beispiel 3: Stabilität und Wiederfindungsraten von 2,4'-Dimethyl-propiophenon und D-Carvon

Die Stabilitäts- und Wiederfindungsraten werden bestimmt durch Lagerung der zu vergleichenden Riechstoffe (2,4'-Dimethyl-propiophenon und D-Carvon, nat.) in der entsprechenden Matrix über einen bestimmten Zeitrahmen.

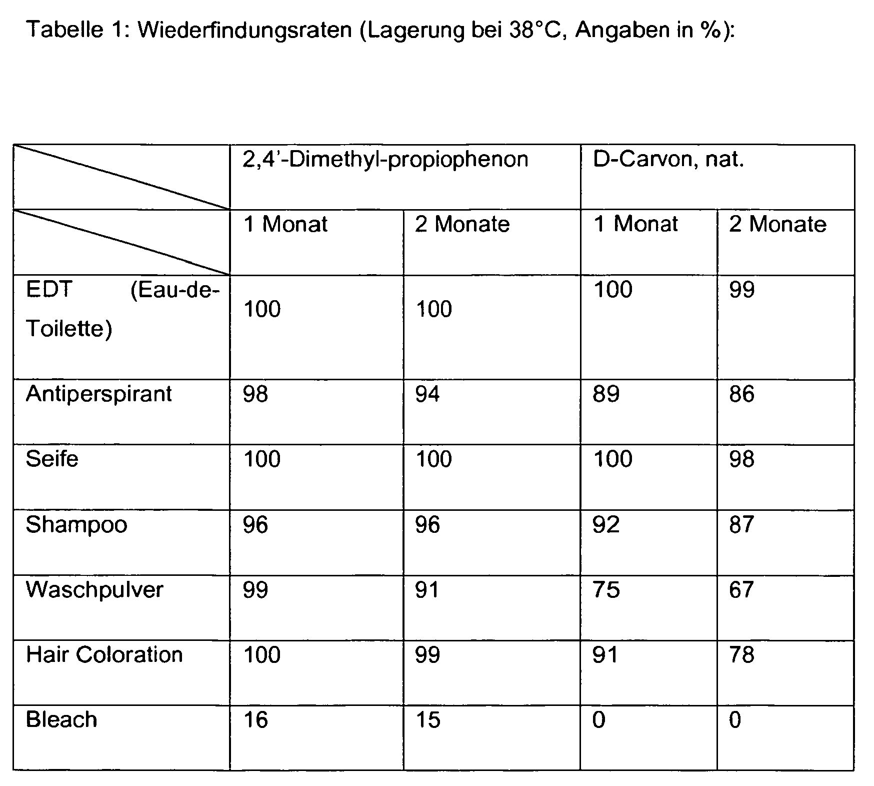

Die Wiederfindungsraten von 2,4'-Dimethyl-propiophenon sind deutlich besser als die von D-Carvon.

Die Rezepturen der o.g. Matrices sind wie folgt:

### EDT (Eau-de-Toilette):

| | |
|---|---|
| Ethanol | 96 % |
| Riechstoff | 4 % |

### Antiperspirant:

| | |
|---|---|
| Demineralisiertes Wasser | 59,4 % |
| Hydroxyethylcellulose | 0,3 % |
| Aluminium Zirconium Pentachlorohydrat | 37,5 % |
| C12-15 Pareth-12 | 1,2 % |
| PEG-40 Hydrogenated Castor oil und Propylenglykol | 0,8 % |
| Riechstoff | 0,8 % |

### Shampoo:

| | |
|---|---|
| Natriumlaurylethersulfat (z.B. Texapon NSO, Fa. Cognis Deutschland GmbH) | 20 % |
| Natriumchlorid | 1,4% |
| Citronensäure | 1,3% |
| Phenoxyethanol, Methyl-, Ethyl-, Butyl-, und Propylparaben | 0,5 % |
| Wasser | 76,3 % |
| Riechstoff | 0,5 % |

### Waschpulver:

| | |
|---|---|
| Lineares Na-Alkylbenzolsulfonat | 8,7 % |
| Ethoxylierter Fettalkohol C12-18 (7 EO) | 4,6 % |
| Na-Seife | 3,2 % |
| Entschäumer | |
| DOW CORNING(R) 2-4248S | |
| POWDERED ANTIFOAM, | |
| Silikonöl auf Zeolith als Trägermaterial | 3,8 % |
| Zeolith 4A | 28,3 % |
| Na-Carbonat | 11,5% |
| Na-Salz eines Copolymers aus Acryl- und Maleinsäure (Sokalan CP5) | 2,4 % |
| Na-Silikat | 3,0 % |
| Carboxymethylcellulose | 1,2 % |
| Dequest 2066 | 2,8% |
| ([[(Phosphonomethyl)imino]bis[(ethylennitrilo)bis (methylen)]]tetrakis-phosphonsäure, Natriumsalz) | |
| Optischer Aufheller | 0,2 % |
| Na-Sulfat | 6,4 % |
| Protease | 0,4 % |
| Natriumperborattetrahydrat | 22,0 % |
| TAED | 1,0% |
| Riechstoff | 0,5 % |

### Hair Coloration / Haarfärbung:

| | |
|---|---|
| Cetylalkohol, Oleylalkohol, Cetearylalkohol und Stearinsäure | 21,0 % |
| Ceteareth-25 | 4,0 % |
| Laureth-8 | 10,0 % |
| Natrium Cetearyl Sulfat | 1,0 % |
| Demineralisiertes Wasser | 49,0 % |
| Polyquaternium-6 | 4,0 % |
| Riechstoff | 0,8 % |
| Ammoniak Lösung (20%ig) | 10,2 % |

### Bleach / Bleiche:

| | |
|---|---|
| Demineralisiertes Wasser | 47,60 % |
| NaOH | 0,5% |
| Dowfax 3B3 (ca. 45%) | 0,8 % |
| Lauryl ether carbonsäure | 0,8 % |
| Stabilisator (Phosphonate) | 0,2 % |
| Na-hypochlorit-Lösung (Gehalt ca. 10,3 %) | 50,0 % |
| Riechstoff | 0,1 % |

Die hier aufgeführten Prozentangaben beziehen sich jeweils, soweit nicht anders erwähnt, auf das Gesamtgewicht des parfümierten Artikels bzw. der Riechstoffkomposition.

### Beispiel 4: Sensorische Daten von 2,4'-Dimethyl-propiophenon im Vergleich mit D-Carvon

**Tabelle 2: Sensorische Daten von 2,4'-Dimethyl-propiophenon im Vergleich mit D-Carvon**

| Test | Skala | 2,4'-Dimethylpropiophenon | D-Carvon, nat. |
|---|---|---|---|
| Geruchsschwellenwert (aus Diethylphthalat) | 1-13 | 4 | 4 |
| Geruchsschwellenwert (aus Luft) | 5-13 | 7,1 | 6,9 |
| Impact (vom Riechstreifen) | 1-9 | 5 | 5 |
| Intensität (an Luft) | 1-9 | 6 | 6 |

Die Bewertung erfolgte jeweils durch ein Sensorik-Panel anhand der angegebenen Skalen, wobei 13 bzw. 9 die bestmöglichen Bewertungen darstellen.

Der Geruchsschwellenwert (aus Diethylphthalat und aus Luft) sowie der Impact (vom Riechstreifen) und die Intensität (an Luft) der verglichenen Riechstoffe sind annähernd gleich zu beurteilen.

### Beispiel 5: Substantivität/Retention von 2,4'-Dimethyl-propiophenon

Wie bereits oben erwähnt , sind für den Einsatz in tensidhaltigen Produkten die Substantivität gegenüber dem Substrat bzw. die Retention am Substrat wichtige anwendungstechnische Anforderungen an die Riechstoffkomposition, insbesondere bei Haaren oder textilen Fasern als Substrat. 2,4'-Dimethyl-propiophenon zeichnet sich, insbesondere für einen blumigen Riechstoff, durch ein hohes Aufziehvermögen und eine hohe Substantivität aus. Dieser Effekt zeigt sich insbesondere auf Substraten wie Haut, Haar und textilen Fasern (z.B. Wolle, Baumwolle, Leinen, synthetische Fasern).

Es wurde ein Performance-Test durchgeführt: Der pH-Wert der Shampoo-Grundmasse (Beschreibung s.o.) lag bei etwa 6. Hieraus wurden 100 mL einer 20 Gew.-%-igen wässrigen Shampoo-Lösung hergestellt. In dieser Shampoo-Lösung wurden 2 Haarsträhnchen gemeinsam für 2 Minuten gewaschen und anschließend 20 Sekunden unter fließendem handwarmen Wasser gespült. Eine Haarsträhne wurde nass in Aluminiumfolie eingepackt und die zweite Haarsträhne mit einem Fön getrocknet. Beide Haarsträhnen wurden von einem Panel auf einer Skala von 0-6 geruchlich beurteilt, wobei 6 die bestmögliche Bewertung darstellt.

**Tabelle 3: Shampoo**

| | 2,4'-Dimethyl-propiophenon | D-Carvon, nat. |
|---|---|---|
| Feuchtes Haar | 2,6 | 2,3 |
| Trockenes Haar | 1,9 | 0,9 |

2,4'-Dimethyl-propiophenon erwies sich also als überlegen.

### Beispiel 6: Blooming-Eigenschaften von 2,4'-Dimethyl-propiophenon

Gesucht werden häufig Riechstoffmischungen mit einer rosenoxid- und herbalartigen Kopfnote, wobei diese - wie oben erwähnt - gleichzeitig ein ausgeprägtes Blooming (Geruch aus einer wässrigen Lösung) aufweisen sollen. Auch insoweit wurde 2,4'-Dimethyl-propiophenon deshalb in einem direkten Performance-Test mit D-Carvon, nat. verglichen (Panel-Bewertung 1-9, wobei 9 die bestmögliche Bewertung darstellt):

| | | 2,4'-Dimethylpropiophenon | D-Carvon, nat. |
|---|---|---|---|
| Blooming | Aus Wasser | 7 | 7 |

Die Verbindung erwies sich demnach ebenfalls als Substanz mit einem starken "Blooming".

Aufgrund der gezeigten positiven Eigenschaften von 2,4'-Dimethyl-propiophenon sind daher besonders bevorzugt zu parfümierende Artikel Waschmittel, Hygiene- oder Pflegeprodukte, insbesondere im Bereich der Körperpflege, der Kosmetik und des Haushalts.

## Patentansprüche

1. Verwendung von 2,4'-Dimethyl-propiophenon als Riechstoff in einer Riechstoffkomposition.

2. Verwendung nach Anspruch 1 zum Vermitteln, Modifizieren und/oder Verstärken einer, zwei, drei oder sämtlicher der Geruchsnoten blumig, rosig, rosenoxidartig und herbalartig, vorzugsweise beider der Geruchsnoten rosenoxidartig und herbalartig.

3. Verwendung von 2,4'-Dimethyl-propiophenon nach einem der vorangehenden Ansprüche, als Bestandteil einer Riechstoffkomposition des Fougere-Typs oder des Rosen-Typs.

4. Riechstoffkomposition enthaltend 2,4'-Dimethyl-propiophenon sowie einen, zwei, drei oder mehr weitere Riechstoffe, wobei das Gewichtsverhältnis der Gesamtmenge an 2,4'-Dimethyl-propiophenon zu der Gesamtmenge an weiteren Riechstoffen im Bereich von 1:1000 bis 1:0,5 liegt, vorzugsweise im Bereich von 1:700 bis 1:1, besonders bevorzugt im Bereich von 1:500 bis 1:10.

5. Riechstoffkomposition nach Anspruch 4, wobei die Menge an 2,4'-Dimethyl-propiophenon ausreicht, eine, zwei, drei oder sämtliche der Geruchsnoten blumig, rosig, rosenoxidartig und herbalartig zu vermitteln, zu modifizieren und/oder zu verstärken, vorzugsweise beide der Geruchsnoten rosenoxidartig und herbalartig.

6. Riechstoffkomposition nach einem der Ansprüche 4 oder 5, wobei die Riechstoffkomposition eine Menge an 2,4'-Dimethyl-propiophenon im Bereich von 0,01 bis 65 Gew.-%, vorzugsweise von etwa 0,1 bis etwa 50 Gew.-%, vorzugsweise von etwa 0,5 bis etwa 30 Gew.-% und besonders bevorzugt von etwa 0,5 bis etwa 25 Gew.-%, umfasst, bezogen auf die Gesamtmenge der Riechstoffkomposition.

7. Riechstoffkomposition nach einem der Ansprüche 4, 5 oder 6, umfassend zumindest einen weiteren Riechstoff, vorzugsweise 2, 3, 4, 5, 6, 7, 8 oder mehr weitere Riechstoffe, wobei der oder die weiteren Riechstoffe ausgewählt sind aus der Gruppe bestehend aus:
Alpha-Hexylzimtaldehyd, 2-Phenoxyethylisobutyrat, Dihydromyrcenol, Methyldihydrojasmonat, 4,6,6,7,8,8-Hexamethyl-1,3,4,6,7,8-hexahydrocyclopenta[g]benzopyran, Tetrahydrolinalool, Ethyllinalool, Benzylsalicylat, 2-Methyl-3-(4-tert-butyl-phenyl)propanal, Zimtalkohol, 4,7-Methano-3a,4,5,6,7,7a-hexahydro-5-indenylacetat und/oder 4,7-Methano-3a,4,5,6,7,7a-hexahydro-6-indenylacetat, Citronellol, Vanillin, Linalylacetat, Styrolylacetat, Octahydro-2,3,8,8-tetramethyl-2-acetonaphthon und/oder 2-Acetyl-1,2,3,4,6,7,8-octahydro-2,3,8,8-tetramethylnaphtalin, Hexylsalicylat, 4-tert.-Butylcyclohexylacetat, 2-tert.-Butylcyclohexylacetat, alpha-Ionon, n-alpha-Methylionon, alpha-Isomethylionon, Coumarin, Terpinylacetat, 2-Phenylethylalkohol, 4-(4-Hydroxy-4-methylpentyl)-3-cyclohexencarboxaldehyd, alpha-Amylzimtaldehyd, Ethylenbrassylat, (E)- und/oder (Z)-3-Methylcyclopentadec-5-enon, 15-Pentadec-11-enolid und/oder 15-Pentadec-12-enolid, 15-Cyclopentadecanolid, 1-(5,6,7,8-Tetrahydro-3,5,5,6,8,8-hexamethyl-2-naphthalenyl)ethanon, 2-Isobutyl-4-methyltetrahydro-2*H*-pyran-4-ol, 2-Ethyl-4-(2,2,3-trimethyl-3-cyclopenten-1-yl)-2-buten-1-ol, cis-3-Hexenylacetat, trans-3-Hexenylacetat, trans-2,cis-6-Nonadienol, 2,4-dimethyl-3-cyclohexencarboxaldehyd, 2,4,4,7-Tetramethyl-oct-6-en-3-on, 2,6-Dimethyl-5-hepten-1-al, Borneol, 3-(3-Isopropylphenyl)butanal, 2-Methyl-3-(3,4-methylendioxyphenyl)propanal, 3-(4-Ethylphenyl)-2,2-dimethylpropanal, 7-Methyl-2*H*-1,5-benzodioxepin-3(4*H*)-on, 3,3,5-Trimethylcyclohexylacetat und 2,5,5-Trimethyl-1,2,3,4,4a,5,6,7-octahydronaphthalin-2-ol.

8. Riechstoffkomposition nach einem der Ansprüche 4, 5, 6 oder 7 mit einer Rosennote, umfassend zumindest einen weiteren Rosenriechstoff, vorzugsweise 2, 3, 4 oder mehr weitere Rosenriechstoffe, wobei der oder die weiteren Rosenriechstoffe ausgewählt sind aus der Gruppe bestehend aus:
Geranylformiat; Geranylacetat; Geranylpropionat; Geranylisobutyrat; Geranylbutyrat; Geranylisovalerianat; Geranyltiglinat; Geranylbenzoat; Citronellylformiat; Citronellylacetat; Citronellylpropionat; Citronellylisobutyrat; Citronellylbutyrat; Citronellylisovalerianat; Citronellyltiglinat; Citronellylbenzoat; Phenylethylalkohol; Phenylacetaldehyd; Phenylacetaaldehyddimethylacetal; Phenylethylformiat; Phenylethylacetat; Phenylethylpropionat; Phenylethylisobutyrat; Phenylethylbutyrat; Phenylethylpivalat; Phenylethylisovalerianat; Phenylethyl-2-ethylbutyrat; Phenylethyltiglinat; Phenylethylbenzoat; Phenylethylphenylacetat; 2-Phenoxyethylisobutyrat; Geranylmethylether; Rosenoxid; Phenylethylmethylether; Phenylethylethylether; Phenylethylisoamylether; 2-Methoxybenzylethylether; alpha-Trichlormethylbenzylacetat; alpha-3,3-Trimethylcyclohexanmethylacetat; 2,4,6-Trimethyl-3-cyclohexenmethanol; N,N-Diethyl-2-ethylhexansäureamid; 3,7-Dimethyloctanol; Geranylaceton; Linalool; 3,7-Dimethyl-1,6-nonadien-3-ol; Nerolidol; Farnesol; 9-Decenol; 9-Decenylacetat; Decanal; 10-Undecenal; 10-Undecenol; Citronellyloxyacetaldehyd; 3,7-Dimethyloctanylacetaldehyd; 2-Methyl-5-phenylpentanol; 2-Methyl-5-phenylpentanal; 3-Methyl-5-phenylpentanol; Benzophenon; Diphenyloxid; Diphenylmethan; alpha-Damascon; beta-Damascon; delta-Damascon; gamma-Damascon; beta-Damascenon; 1-(2,4,4-Trimethyl-2-cyclohexenyl)-2-buten-1-on; Rosenöl; Rosenabsolue; Geraniumöl, Geraniol, Nerol, 2-Phenylethylalkohol.

9. Riechstoffkomposition nach einem der Ansprüche 4, 5, 6 oder 7 mit einer Fougèrenote, umfassend zumindest einen weiteren herbal-krautigen Riechstoff, vorzugsweise 2, 3, 4 oder mehr weitere herbal-krautige Riechstoffe, wobei der oder die weiteren herbal-krautigen Riechstoffe ausgewählt sind aus der Gruppe bestehend aus:
4,7-Methano-3a,4,5,6,7,7a-hexahydro-5 (oder -6) -indenylacetate; beta-Orcincarbonsäure-methylester; Terpinylacetat; Terpineol; Terpinenol-4; Linalool; Campher; 1,8-Cineol; Lavandulol; Lavandulylacetat; 1-Octen-3-ol; Caryophyllen; Farnesen; 2-Butyl-4,4,6-trimethyl-1,3-dioxan.

10. Parfümierter Artikel, umfassend eine Riechstoffkomposition nach einem der Ansprüche 4 bis 9, wobei der Artikel ausgewählt ist aus der Gruppe bestehend aus: Parfüm-Extraits, Eau de Parfums, Eau de Toilettes, Rasierwässer, Eau de Colognes, Pre-shave-Produkte, Splash-Colognes, parfümierte Erfrischungstücher, Parfüms für saure, alkalische und neutrale Reinigungsmittel, Waschmittel, Waschtabletten, Desinfektionsmitteln, sowie von Luftverbesserern, Aerosolsprays, Wachse und Polituren, sowie Körperpflegemitteln, Badeölen, kosmetischen Emulsionen, Haarpflegeprodukten, Deodorantien und Antiperspirantiens, Produkten der dekorativen Kosmetik sowie von Kerzen, Lampenölen, Räucherstäbchen, Insektiziden, Repellentien, Treibstoffen.

11. Verwendung von 2,4'-Dimethyl-propiophenon oder einer 2,4'-Dimethyl-propiophenon enthaltenden Mischung als Mittel zum Versehen von (a) Haaren oder (b) textilen Fasern mit einer, zwei, drei oder sämtlichen der Geruchsnoten blumig, rosig, rosenoxidartig und herbalartig, vorzugsweise mit einer rosenoxidartigen und herbalartigen Geruchsnote.

12. Verwendung von 2,4'-Dimethyl-propiophenon als Mittel zum Erhöhen der Substantivität und/oder Retention einer Riechstoffkomposition.

13. Verfahren zur Herstellung einer Riechstoffkomposition nach einem der Ansprüche 4 bis 9, umfassend den folgenden Schritt:
- Vermischen von 2,4'-Dimethyl-propiophenon mit der oder den weiteren Riechstoffen sowie gegebenenfalls weiteren Bestandteilen der Riechstoffkomposition.

14. Verfahren nach Anspruch 13, wobei das 2,4'-Dimethyl-propiophenon in einer Menge eingesetzt wird, die ausreicht, um in der Riechstoffkomposition eine, zwei, drei oder sämtliche der Geruchsnoten blumig, rosig, rosenoxidartig und herbalartig, vorzugsweise beide der Geruchsnoten rosenoxidartig und herbalartig, zu vermitteln, zu modifizieren und/oder zu verstärken.

## Claims

1. Use of 2,4'-dimethyl propiophenone as an aroma in an aroma composition.

2. Use according to claim 1 to impart, modify and/or strengthen one, two, three or all of the scent notes floral, rose, rose oxide and herbal, preferably the two scent notes rose oxide and herbal.

3. Use of 2,4'-dimethyl propiophenone according to one of the preceding claims as a constituent of an aroma composition of the fougère type or rose type.

4. Aroma composition containing 2,4'-dimethyl propiophenone and one, two, three or more further aromas, wherein the weight ratio of the total amount of 2,4'-dimethyl propiophenone to the total amount of further aromas is in the range from 1:1000 to 1:0.5, preferably in the range from 1:700 to 1:1, particularly preferably in the range from 1:500 to 1:10.

5. Aroma composition according to claim 4, wherein the amount of 2,4'-dimethyl propiophenone is sufficient to impart, to modify and/or to strengthen one, two, three or all of the scent notes floral, rose, rose oxide and herbal, preferably the two scent notes rose oxide and herbal.

6. Aroma composition according to one of claims 4 or 5, wherein the aroma composition comprises an amount of 2,4'-dimethyl propiophenone in the range from 0.01 to 65 wt.%, preferably from approximately 0.1 to approximately 50 wt.%, preferably from approximately 0.5 to approximately 30 wt.% and particularly preferably from approximately 0.5 to approximately 25 wt.%, relative to the total amount of the aroma composition.

7. Aroma composition according to one of claims 4, 5 or 6, comprising at least one further aroma, preferably 2, 3, 4, 5, 6, 7, 8 or more further aromas, wherein the further aroma(s) are selected from the group consisting of:
alpha-hexyl cinnamaldehyde, 2-phenoxyethyl isobutyrate, dihydromyrcenol, methyl dihydrojasmonate, 4,6,6,7,8,8-hexamethyl-1,3,4,6,7,8-hexahydrocyclopenta[g]benzopyrane, tetrahydrolinalool, ethyl linalool, benzyl salicylate, 2-methyl-3-(4-tert-butyl phenyl)propanol, cinnamyl alcohol, 4,7-methano-3a,4,5,6,7,7a-hexahydro-5-indenyl acetate and/or 4,7-methano-3a,4,5,6,7,7a-hexahydro-6-indenyl acetate, citronellol, vanillin, linalyl acetate, styrolyl acetate, octahydro-2,3,8,8-tetramethyl-2-acetonaphthone and/or 2-acetyl-1,2,3,4,6,7,8-octahydro-2,3,8,8-tetamethyl naphthalene, hexyl salicylate, 4-tert-butyl cyclohexyl acetate, 2-tert-butyl cyclohexyl acetate, alpha-ionone, n-alpha-methyl ionone, alpha-isomethyl ionone, coumarin, terpinyl acetate, 2-phenylethyl alcohol, 4-(4-hydroxy-4-methylpentyl)-3-cyclohexene carboxaldehyde, alpha-amyl cinnamaldehyde, ethylene brassylate, (E)- and/or (Z)-3-methyl cyclopentadec-5-enone, 15-pentadec-11-enolide and/or 15-pentadec-12-enolide, 15-cyclopentadecanolide, 1-(5,6,7,8-tetrahydro-3,5,5,6,8,8-hexamethyl-2-naphthalenyl)ethanone, 2-isobutyl-4-methyl tetrahydro-2H-pyran-4-ol, 2-ethyl-4-(2,2,3-trimethyl-3-cyclopenten-1-yl)-2-buten-1-ol, cis-3-hexenyl acetate, trans-3-hexenyl acetate, trans-2,cis-6-nonadienol, 2,4-dimethyl-3-cyclohexene carboxaldehyde, 2,4,4,7-tetramethyl oct-6-en-3-one, 2,6-dimethyl-5-hepten-1-al, borneol, 3-(3-isopropylphenyl)butanal, 2-methyl-3-(3,4-methylene dioxyphenyl)propanal, 3-(4-ethylphenyl)-2,2-dimethyl propanal, 7-methyl-2H-1,5-benzodioxepin-3(4H)-one, 3,3,5-trimethyl cyclohexyl acetate and 2,5,5-trimethyl-1,2,3,4,4a,5,6,7-octahydronaphthalen-2-ol.

8. Aroma composition according to one of claims 4, 5, 6 or 7 having a rose note, comprising at least one further rose aroma, preferably 2, 3, 4 or more further rose aromas, wherein the further rose aroma(s) are selected from the group consisting of:
geranyl formate; geranyl acetate; geranyl propionate; geranyl isobutyrate; geranyl butyrate; geranyl isovalerianate; geranyl tiglinate; geranyl benzoate; citronellyl formate; citronellyl acetate; citronellyl propionate; citronellyl isobutyrate; citronellyl butyrate; citronellyl isovalerianate; citronellyl tiglinate; citronellyl benzoate; phenylethyl alcohol; phenyl acetaldehyde; phenyl acetaldehyde dimethyl acetal; phenylethyl formate; phenylethyl acetate; phenylethyl propionate; phenylethyl isobutyrate; phenylethyl butyrate; phenylethyl pivalate; phenylethyl isovalerianate; phenylethyl-2-ethylbutyrate; phenylethyl tiglinate; phenylethyl benzoate; phenylethyl phenyl acetate; 2-phenoxyethyl isobutyrate; geranyl methyl ether; rose oxide; phenylethyl methyl ether, phenylethyl ethyl ether; phenylethyl isoamyl ether; 2-methoxybenzyl ethyl ether, alpha-trichloromethyl benzyl acetate; alpha-3,3-trimethyl cyclohexane methyl acetate; 2,4,6-trimethyl-3-cyclohexene methanol; N,N-diethyl-2-ethyl hexanoic acid amide; 3,7-dimethyl octanol; geranyl acetone; linalool; 3,7-dimethyl-1,6-nonadien-3-ol; nerolidol; farnesol; 9-decenol; 9-decenyl acetate; decanal; 10-undecenal; 10-undecenol; citronellyl oxyacetaldehyde; 3,7-dimethyl octanyl acetaldehyde; 2-methyl-5-phenyl pentanol; 2-methyl-5-phenyl pentanal; 3-methyl-5-phenyl pentanol; benzophenone; diphenyl oxide; diphenyl methane; alpha-damascone; beta-damascone; delta-damascone; gamma-damascone; beta-damascenone; 1-(2,4,4-trimethyl-2-cyclohexenyl)-2-buten-1-one; rose oil; rose absolute; geranium oil; geraniol; nerol; 2-phenylethyl alcohol.

9. Aroma composition according to one of claims 4, 5, 6 or 7 having a fougère note, comprising at least one further herbal/herbaceous aroma, preferably 2, 3, 4 or more further herbal/herbaceous aromas, wherein the further herbal/herbaceous aroma(s) are selected from the group consisting of:
4,7-methano-3a,4,5,6,7,7a-hexahydro-5- (or -6) -indenyl acetates; beta-orcinol carboxylic acid methyl ester; terpinyl acetate; terpineol; terpinenol-4; linalool; camphor; 1,8-cineole; lavandulol; lavandulyl acetate; 1-octen-3-ol; caryophyllene; famesene; 2-butyl-4,4,6-trimethyl-1,3-dioxane.

10. Perfumed item comprising an aroma composition according to one of claims 4 to 9, wherein the item is selected from the group consisting of: perfume extracts, eau de parfums, eau de toilettes, aftershaves, eau de colognes, preshave products, splash colognes, perfumed refreshing wipes, perfumes for acid, alkaline and neutral cleaning agents, detergents, detergent tablets, disinfectants and for air fresheners, aerosol sprays, waxes and polishes, as well as body care products, bath oils, cosmetic emulsions, hair care products, deodorants and antiperspirants, decorative cosmetic products and also candles, lamp oils, incense sticks, insecticides, repellents, fuels.

11. Use of 2,4'-dimethyl propiophenone or a mixture containing 2,4'-dimethyl propiophenone as an agent for imparting one, two, three or all of the scent notes floral, rose, rose oxide and herbal, preferably a rose oxide and herbal floral note, to (a) hair or (b) textile fibres.

12. Use of 2,4'-dimethyl propiophenone as an agent for increasing the substantivity and/or retention of an aroma composition.

13. Method for preparing an aroma composition according to one of claims 4 to 9, comprising the following step:
- Mixing of 2,4'-dimethyl propiophenone with the further aroma(s) and optionally further constituents of the aroma composition.

14. Method according to claim 13, wherein the 2,4'-dimethyl propiophenone is used in an amount sufficient to impart, to modify and/or to strengthen one, two, three or all of the scent notes floral, rose, rose oxide and herbal, preferably the two scent notes rose oxide and herbal, in the aroma composition.

## Revendications

1. Utilisation de 2,4'-diméthyl-propiophénone comme matière odorante dans une composition de matières odorantes.

2. Utilisation selon la revendication 1 pour conférer, modifier et/ou renforcer une, deux, trois ou toutes les notes olfactives fleur, rose, oxyde de rose et herbe, de préférence des deux notes olfactives oxyde de rose et herbe.

3. Utilisation de 2,4'-diméthyl-propiophénone selon l'une quelconque des revendications précédentes comme composant d'une composition de matières odorantes du type fougère ou du type rose.

4. Composition de matières odorantes contenant de la 2,4'-diméthyl-propiophénone ainsi qu'une, deux, trois ou plusieurs autres matières odorantes, sachant que le rapport pondéral de la quantité totale de 2,4'-diméthyl-propiophénone à la quantité totale des autres matières odorantes est compris dans l'intervalle de 1:1000 à 1:0,5, de préférence dans l'intervalle de 1:700 à 1:1, de façon particulièrement préférée dans l'intervalle de 1:500 à 1:10.

5. Composition de matières odorantes selon la revendication 4, dans laquelle la quantité de 2,4'-diméthylpropiophénone suffit pour conférer, modifier et/ou renforcer une, deux, trois ou toutes les notes olfactives fleur, rose, oxyde de rose et herbe, de préférence les deux notes olfactives oxyde de rose et herbe.

6. Composition de matières odorantes selon l'une quelconque des revendications 4 ou 5, dans laquelle la composition de matières odorantes comprend une quantité de 2,4'-diméthyl-propiophénone dans l'intervalle de 0,01 à 65 % en poids, de préférence d'environ 0,1 à environ 50 % en poids, de préférence d'environ 0,5 à environ 30 % en poids et de façon particulièrement préférée d'environ 0,5 à environ 25 % en poids, rapportés à la quantité totale de la composition de matières odorantes.

7. Composition de matières odorantes selon l'une quelconque des revendications 4, 5 ou 6, comprenant au moins une autre matière odorante, de préférence 2, 3, 4, 5, 6, 7, 8 ou plusieurs autres matières odorantes, la ou les autres matières odorantes étant choisies parmi le groupe constitué de :
aldéhyde alpha-hexylcinnamique, 2-phénoxyéthylisobutyrate, dihydromyrcénol, méthyldihydrojasmonate, 4,6,6,7,8,8-hexaméthyl-1,3,4,6,7,8-exahydrocyclopenta[g]benzopyrane, tétrahydrolinalool, éthyllinalool, benzylsalicylate, 2-méthyl-3-(4-tert-butyl-phényl)propanal, alcool cinnamique, 4,7-méthano-3a,4,5,6,7,7a-hexahydro-5-indénylacétate et/ou 4,7-méthano-3a,4,5,6,7,7a-hexahydro-6-indénylacétate, citronellol, vanilline, linalylacétate, styrolylacétate, octahydro-2,3,8,8-tétraméthyl-2-acétonaphthone et/ou 2-acétyl-1,2,3,4,6,7,8-octahydro-2,3,8,8-tétraméthylnaphtalène, hexylsalicylate, 4-tert.-butylcyclohexylacétate, 2-tert.-butylcyclohexylacétate, alpha-ionone, n-alpha-méthylionone, alpha-isométhylionone, coumarine, terpinylacétate, alcool 2-phényléthylique, 4-(4-hydroxy-4-méthylpentyl)-3-cyclohexènecarboxaldéhyde, aldéhyde alpha-amylcinnamique, éthylenbrassylate, (E)- et/ou (Z)-3-méthylcyclopentadéc-5-énone, 15-pentadéc-11-énolide et/ou 15-pentadéc-12-énolide, 15-cyclopentadécanolide, 1-(5,6,7,8-tétrahydro-3,5,5,6,8,8-hexaméthyl-2-naphthalényl)éthanone, 2-isobutyl-4-méthyltétrahydro-2H-pyran-4-ol, 2-éthyl-4(2,2,3-triméthyl-3-cyclopentén-1-yl)-2-butén-1-ol, cis-3-hexénylacétate, trans-3-hexénylacétate, trans-2,cis-6-nonadiénol, 2,4-diméthyl-3-cyclohexènecarboxaldéhyde, 2,4,4,7-tétraméthyl-oct-6-én-3-one, 2,6-diméthyl-5-heptén-1-al, bornéol, 3-(3-isopropylphényl)butanal, 2-méthyl-3-(3,4-méthylènedioxyphényl)propanal, 3-(4-éthylphényl)-2,2-diméthylpropanal, 7-méthyl-2H-1,5-benzodioxépine-3(4H)-one, 3,3,5-triméthylcyclohexylacétate et 2,5,5-triméthyl-1,2,3,4,4a,5,6,7-octahydronaphthalén-2-ol.

8. Composition de matières odorantes selon l'une quelconque des revendications 4, 5, 6 ou 7 avec une note de rose, comprenant au moins une autre matière odorante de rose, de préférence 2, 3, 4 ou plusieurs autres matières odorantes de rose, la ou les autres matières odorantes de rose étant choisies parmi le groupe constitué de :
géranylformiate; géranylacétate; géranylpropionate; géranylisobutyrate; géranylbutyrate; géranylisovalérianate ; géranyltiglinate ; géranylbenzoate ; citronellylformiate ; citronellylacétate; citronellylpropionate; citronellylisobutyrate ; citronellylbutyrate; citronellylisovalérianate ; citronellyltiglinate; citronellylbenzoate; alcool phényléthylique; phénylacétaldéhyde; phénylacétaldéhydediméthylacétal; phényléthylformiate; phényléthylacétate ; phényléthylpropionate; phényléthylisobutyrate; phényléthylbutyrate; phényléthylpivalate; phényléthylisovalérianate; phényléthyl-2-éthylbutyrate; phényléthyltiglinate; phényléthylbenzoate; phényléthylphénylacétate 2-phénoxyéthylisobutyrate; géranylméthyléther, oxyde de rose; phényléthylméthyléther; phényléthyléthyléther; phényléthylisoamyléther; 2-méthoxybenzyléthyléther, alpha-trichlorométhylbenzylacétate; alpha-3,3-triméthylcyclohexaneméthylacétate; 2,4,6-triméthyl-3-cyclohexèneméthanol; amide N,N-diéthyl-2-éthylhexanoïque; 3,7-diméthyloctanol; géranylacétone; linalool; 3,7-diméthyl-1,6-nonadién-3-ol; nérolidol; famésol; 9-décénol; 9-décénylacétate; décanal; 10-undécénal; 10-undécénol; citronellyloxyacétaldéhyde; 3,7-diméthyloctanylacétaldéhyde; 2-méthyl-5-phénylpentanol; 2-méthyl-5-phénylpentanal; 3-méthyl-5-phénylpentanol ; benzophénone; diphényloxyde; diphénylméthane; alpha-damascone; beta-damascone; delta-damascone; gamma-damascone; beta-damascénone; 1-(2,4,4-triméthyl-2-cyclohexényl)-2-butén-1-one; huile de rose; rose absolue; huile de géranium, géraniol, nérol, alcool 2-phényléthylique.

9. Composition de matières odorantes selon l'une quelconque des revendications 4, 5, 6 ou 7 avec une note de fougère, comprenant au moins une autre matière odorante d'herbe ou de plante médicinale, de préférence 2, 3, 4 ou plusieurs autres matières odorantes d'herbe ou de plante médicinale, sachant que la ou les autres matières odorantes d'herbe ou de plante médicinale sont choisies parmi le groupe constitué de :
4,7-méthano-3a,4,5,6,7,7a-hexahydro-5 (ou -6) -indénylacétate; ester méthylique de l'acide beta-orcine carboxylique; terpinylacétate; terpinéol; terpinénol-4; linalool; camphre; 1,8-cinéol; lavandulol; lavandulylacétate; 1-octén-3-ol; caryophyllène; farnesène; 2-butyl-4,4,6-triméthyl-1,3-dioxane.

10. Article parfumé, comprenant une composition de matières odorantes selon l'une quelconque des revendications 4 à 9, l'article étant choisi parmi le groupe constitué de : extraits de parfum, eaux de parfums, eaux de toilettes, lotions après-rasage, eaux de Cologne, produits avant-rasage, Splash-Colognes, serviettes rafraîchissantes parfumées, parfums pour produits de nettoyage acides, alcalins et neutres, lessives, comprimés de lavage, désinfectants, ainsi que produits d'amélioration de l'air, sprays d'aérosols, cires et produits à polir, acides, alcalins et neutres, lessives, comprimés de lavage, désinfectants, ainsi que produits d'amélioration de l'air, sprays d'aérosols, cires et produits à polir, ainsi que produits d'hygiène corporelle, huiles de bain, émulsions cosmétiques, produits d'hygiène capillaire, déodorants et antitranspirants, produits de cosmétique décorative ainsi que bougies, huiles de lampe, bâtonnets d'encens, insecticides, répulsifs, carburants.

11. Utilisation de 2,4'-diméthyl-propiophénone ou d'un mélange contenant de la 2,4'-diméthylpropiophénone comme moyen pour conférer (a) à des cheveux ou (b) à des fibres textiles une, deux, trois ou toutes les notes olfactives fleur, rose, oxyde de rose ou herbe, de préférence une note olfactive oxyde de rose ou herbe.

12. Utilisation de la 2,4'-diméthyl-propiophénone comme moyen pour accroître la substantivité et/ou la rétention d'une composition de matières odorantes.

13. Procédé de préparation d'une composition de matières odorantes selon l'une quelconque des revendications 4 à 9, comprenant l'étape suivante :
- mélangeage de 2,4'-diméthyl-propiophénone avec la ou les autres matières odorantes ainsi que le cas échéant d'autres composants de la composition de matières odorantes.

14. Procédé selon la revendication 13, la 2,4'-diméthyl-propiophénone étant utilisée en une quantité qui suffit pour conférer, modifier et/ou renforcer un, deux, trois ou toutes les notes olfactives fleur, rose, oxyde de rose et herbe, de préférence des deux notes olfactives oxyde de rose et herbe dans la composition de matières odorantes
